# EUROPEAN PATENT SPECIFICATION

(11) **EP 1 909 824 B1**
(45) Date of publication and mention of the grant of the patent: **18.05.2011**
(21) Application number: 06740736.1
(22) Date of filing: 06.04.2006
(51) Int. Cl.: A61K 38/22

(54) **PHARMACEUTICAL FORMULATIONS COMPRISING INCRETIN PEPTIDE AND APROTIC POLAR SOLVENT**
PHARMAZEUTISCHE FORMULIERUNGEN MIT INCRETIN-PEPTID UND APROTISCH-POLAREM LÖSUNGSMITTEL
FORMULATIONS PHARMACEUTIQUES COMPRENANT UN PEPTIDE INCRETINE ET UN SOLVANT POLAIRE APROTIQUE

(30) Priority: 08.04.2005 US 669353 P
(43) Date of publication of application: 16.04.2008
(73) Proprietor: Amylin Pharmaceuticals, Inc., San Diego CA 92121 (US)
(72) Inventor: JENNINGS, Robert N. Jr., San Diego, California 92121 (US); ONG, John T.H., San Diego, California 92121 (US); RHODES, Christopher A., San Diego, California 92121 (US); STETSKO, Gregg, San Diego, California 92121 (US); PRESTRELSKI, Steven J., San Diego, California 92121 (US)
(74) Representative: UEXKÜLL & STOLBERG
(86) International application number: PCT/US2006/013073
(87) International publication number: WO 2006/110551

(56) References cited:
- US-A- 5 766 620
- US-A- 5 932 547
- HUDSON F M; ANDERSEN N H: "Exenatide: NMR/CD evaluation of the medium dependence of conformation and aggregation state" BIOPOLYMERS, vol. 76, no. 4, 3 September 2004 (2004-09-03), pages 298-308, XP002422189
- KEMP D M ET AL: "Synergistic effect of dimethyl sulfoxide on glucagon-like peptide 1 (GLP-1) - stimulated insulin secretion and gene transcription in INS-1 cells: characterization and implications" BIOCHEMICAL PHARMACOLOGY, PERGAMON, OXFORD, GB, vol. 64, no. 4, August 2002 (2002-08), pages 689-697, XP002975842 ISSN: 0006-2952

## Description

### FIELD OF THE INVENTION

The present invention relates to pharmaceutical formulations, and more particularly to pharmaceutical formulations of peptides and proteins with improved stability, namely, comprising an incretin or incretin mimetic peptide, at least one aprotic, polar solvent and at least one stabilizing excipient selected from the group consisting of water, a sugar, and a sugar alcohol.

### BACKGROUND

Treatment of disease by prolonged delivery of an active agent at a controlled rate has been a goal in the drug delivery field. Various approaches have been taken toward delivering the active agents.

Approaches have involved the use of implantable diffusional systems and implantable infusion pumps for delivering drugs, *e.g*., by intravenous, intra-arterial, subcutaneous; intrathecal, intraperitoneal, intraspinal and epidural pathways. The pumps are usually surgically inserted into a subcutaneous pocket of tissue in the lower abdomen. Systems for pain management, chemotherapy and insulin delivery are described in the BBI Newsletter, Vol. 17, No. 12, pages 209-211, December 1994.

One approach involves osmotically driven devices such as those described in U.S. Pat. Nos. 3,987,790, 4,865,845, 5,057,318, 5,059,423, 5,112,614, 5,137,727, 5,234,692 and 5,234,693. These devices can be implanted into an animal to release the active agent in a controlled manner for a predetermined administration period. In general, these devices operate by imbibing fluid from the outside environment and releasing corresponding amounts of the active agent.

Other exemplary implantable devices are taught in U.S. Pat Nos. 5,034,229, 5,057,318, 5,110,596, and 5,782,396. Yet other exemplary implantable devices include regulator-type implantable pumps that provide constant flow, adjustable flow, or programmable flow of beneficial agent formulations, which are available from, for example, Codman of Raynham, Mass., Medtronic of Minneapolis, Minn., and Tricumed Medinzintechnik GmbH of Germany. Further examples of implantable devices are described in U.S. Pat. Nos. 6,395,292, 6,283,949, 5,976,109, 5,836,935, 5,511,355.

Controlled delivery of a beneficial agent from an implantable device over prolonged periods of time has several potential advantages. For instance, use of implantable delivery devices generally assures patient compliance, as implantable devices are not easily tampered with by the patient and can be designed to provide therapeutic doses of beneficial agent over periods of weeks, months, or even years without patient input. Moreover, because an implantable device may be placed only once during its functional life, implantable devices may offer reduced site irritation, fewer occupational hazards for patients and practitioners, reduced waste disposal hazards, decreased costs, and increased efficacy when compared to other parenteral administration techniques, such as injections, that require multiple administrations over relatively short time intervals. However, providing controlled delivery of beneficial agents from implantable devices presents several technical challenges, and controlled delivery of peptides, polypeptides, proteins and other proteinaceous substances over sustained periods of time from implantable devices has proven particularly difficult.

In order to deliver a beneficial agent from an implanted device at a controlled rate over a prolonged period of time (*i.e.,* a period of weeks, months, or years), the beneficial agent must be formulated such that it is stable at ambient and physiological temperatures. Proteins are naturally active in aqueous environments, and protein formulations have generally been aqueous solutions. However, proteins are typically only marginally stable in aqueous formulations for long durations of time, and aqueous pharmaceutical preparations of proteins have often required refrigeration or exhibited short shelf-lives at ambient or physiological temperatures.

Proteins can degrade via a number of mechanisms, including deamidation, oxidation, hydrolysis, disulfide interchange, and racemization. Further, water acts as a plasticizer, which facilitates unfolding of protein molecules and irreversible molecular aggregation. Therefore, in order to provide a protein formulation that is stable over time at ambient or physiological temperatures, a non-aqueous or substantially non-aqueous protein formulation is generally required.

Reduction of aqueous protein formulations to dry powdered formulations is one way to increase the stability of pharmaceutical protein formulations. For example, protein formulations can be dried using various techniques, including spray-drying, lyophilization or freeze-drying, and dessication. The dry powder protein formulations achieved by such techniques exhibit significantly increased stability over time at ambient or even physiological temperatures. However, where a flowable protein formulation is required, such as in an implantable delivery device, dry powder protein formulations alone are of limited use.

In order to provide stable, flowable protein formulations, some have suggested using solution formulations of peptides in non-aqueous, aprotic, polar solvents. Such formulations have shown to be stable at elevated temperatures for long periods of time. However, solvent based formulations are not suitable for all proteins because many proteins have low solubility in solvents that are suitable for parenteral administration. As the solubility of protein in the solvent decreases, the amount of formulation required to deliver a given protein dose will increase, and though relatively large volumes of low concentration solutions of protein may be useful for delivery by injection, due to size constraints, implantable delivery devices generally require relatively high concentration protein formulations capable of delivering therapeutic levels of protein at low flow rates over prolonged periods of time.

Thus, it is desirable to develop formulations that provide the stability and delivery characteristics necessary to deliver beneficial agents, such as peptides and proteins, from an implantable delivery device at a controlled rate over a prolonged period of time.

### SUMMARY OF THE INVENTION

To address such needs and others, provided herein are stable pharmaceutical formulations and uses thereof. The formulations generally comprise an incretin or incretin mimetic peptide, such as an exendin peptide, at least one aprotic, polar solvent, and optionally one or more stabilizing excipients selected from the group consisting of water, a sugar, and a sugar alcohol. The peptide is stabilized in the formulation so as to allow for long-term storage and/or delivery over a prolonged period of time.

As such, one aspect is directed to the use of aprotic, polar solvents, such as DMSO, to stabilize peptide formulations against both chemical and physical degradation. It has been found that the aprotic, polar solvent improves the overall stability of incretin and incretin mimetic peptides in a wide range of formulation conditions, including high concentrations and elevated or non-refrigerated temperatures, thus making possible the long-term storage of such peptides at elevated or room temperature, as well as the delivery of such peptides in long-term devices that would not otherwise be feasible, such as pen style injection devices or pump style delivery devices.

Further disclosed are methods for improving the long-term stability and achieving extended delivery of therapeutically active peptides or proteins using a suitable reservoir from which the formulated peptide may be pumped or metered out at a controlled rate. The reservoir may be implanted under the skin (e.g., as an implantable pump device) or may be external to the body and either attached or not attached (e.g., as a pen style injection device or external pump device). The peptide may be formulated in a manner to provide stability at physiologic temperatures for the duration of therapeutic exposure, and may provide a supply of therapeutically active material for up to 2 years.

These and other aspects of the invention will become apparent to one of skill in the art.

### SUMMARY

A stable pharmaceutical formulation as described in the claims, comprising an incretin or incretin mimetic peptide and at least one aprotic, polar solvent is provided. Examples of incretins or incretin mimetic peptides are glucagon-like-peptide 1 (GLP-1) peptides, exendin peptides and analogs thereof. In some embodiments, the exendin peptide is exendin-4 or an analog thereof. Non-limiting examples of aprotic, polar solvents are dimethylsulfoxide (DMSO) dimethylformamide (DMF), ethyl acetate, n-methyl pyrrolidone (NMP), dimethylacetamide (DMA), propylene carbonate, and mixtures thereof. In some embodiments, the aprotic, polar solvent is DMSO.

The pharmaceutical formulation further comprises at least one stabilizing excipient, additive or solvent selected from the group consisting of water, a sugar, and a sugar alcohol. In some embodiments, the stabilizing excipient, additive or solvent is capable of depressing the freezing point of the aprotic, polar solvent to about 0°C or below. Stabilizing excipients, additives or solvents capable of depressing the freezing point of the aprotic, polar solvent are water, sugars, and sugar alcohols. In some embodiments, the stable aprotic, polar solvent is DMSO, the stabilizing excipient, additive or solvent capable of depressing the freezing point of the aprotic, polar solvent is water, and the water and DMSO form a co-solvent comprising about 10% w/w water (0.67 mole fraction DMSO). In some embodiments, the stabilizing excipient is capable of stabilizing the conformation of the incretin or incretin mimetic peptide.

In some embodiments, the incretin peptide comprises one or more amino acid residues selected from the group consisting of asparagine, glutamine, aspartic acid, glutamic acid, methionine, cysteine, tryptophan, tyrosine, histidine, lysine, and arginine, and the aprotic, polar solvent, the stabilizing excipient, or both stabilize the amino acid residue from degradation. In some embodiments, the amino acid residue is asparagine or glutamine, and the aprotic, polar solvent, the stabilizing excipient, or both stabilize the amino acid residue against degradation by reducing or preventing the formation of cyclic imide or other degradation products of asparagine and glutamine amino acid residues.

In some embodiments, the stable pharmaceutical formulation further comprises at least one non-aqueous aprotic solvent. Examples of non-aqueous protic solvents are polyethylene glycol (PEG), propylene glycol (PG), polyvinylpyrrolidone (PVP), methoxypropylene glycol (MPEG), glycerol, glycofurol, and mixtures thereof.

In some embodiments, the stable pharmaceutical formulation further comprises a buffer. In some embodiments, the buffer is an acetate buffer.

In some embodiments, the stable pharmaceutical formulation further comprises an antioxidant. Examples of antioxidants include ascorbic acid, cysteine, methionine, monothioglycerol, sodium thiosulphate, sulfites, BHT, BHA, ascorbyl palmitate, propyl gallate, and Vitamin E.

In some embodiments, the stable pharmaceutical formulation further comprises a chelator. Examples of chelators are EDTA, tartaric acid and salts thereof, glycerin, and citric acid and salts thereof.

In some embodiments, the stable pharmaceutical formulation further comprises a sugar, a sugar alcohol, or a non-aqueous solvent. Examples of non-aqueous solvents are ethanol, glycerin, propylene glycol, and polyethylene glycol.

In some embodiments, the stable pharmaceutical formulation further comprises a preservative. Examples of preservatives are benzyl alcohols, methyl parabens and propyl parabens.

In some embodiments, the stable pharmaceutical formulation further comprises a thermo-responsive polymer that does not gel at a temperature from about 30°C to about 37°C.

In some embodiments, the incretin or incretin mimetic is complexed with zinc to form a zinc complex. In some embodiments, the zinc complex comprises a GLP-1 or an analog thereof. In some embodiments, the zinc complex comprises an exendin or an analog thereof. In some embodiments, the zinc complex comprises an exendin-4 zinc complex. In some embodiments, the zinc complex is dispersed in the solvent.

In some embodiments, the stable pharmaceutical formulation has a viscosity of from about 0.25 cP to about 1,000,000 cP.

In some embodiments, the stable pharmaceutical formulation has a pH at or below the pI of the incretin or incretin mimetic. In some embodiments, the stable pharmaceutical formulation has a pH of from about pH 4.0 to about pH 7.5. In some embodiments, the stable pharmaceutical formulation has a pH of from about pH 4.0 to about pH 6.0. In some embodiments, the stable pharmaceutical formulation has a pH of about 4.5.

In some embodiments, the incretin or incretin mimetic is present at a concentration from about 0.1 mg/ml up to the solubility limit of the incretin peptide in the formulation. In some embodiments, the incretin or incretin mimetic is present at a concentration from about 1 mg/ml to about 100 mg/ml.

In some embodiments, the stable pharmaceutical formulation is further lyophilized. In some embodiments, the lyophilized formulation is reconstituted prior to use.

In some embodiments, the peptide is lyophilized from a solution with a pH ranging from about pH 4.0 to about pH 7.5. In some embodiments, the peptide is lyophilized from a solution with a pH ranging from about pH 4.0 to about pH 6.0. In some embodiments, the peptide is lyophilized from a solution with a pH of about pH 4.5.

Also provided is a pharmaceutical formulation as described herein, for use in the treatment of a disease, condition or disorder that may be treated, alleviated or prevented by administering an incretin or an incretin mimetic.

In some embodiments, the disease, condition or disorder comprises glucose intolerance or diabetes mellitus. In some embodiments, the disease, condition or disorder is diabetes mellitus. In some embodiments, the disease, condition or disorder is type 2 diabetes.

In some embodiments, the administration is parenteral administration. In some embodiments, the administration is continuous administration. In some embodiments, the administration is accomplished via use of an implantable or attachable pump drug delivery device. In some embodiments, the administration is continuous for a period ranging from about 1 month to about 6 months. In some embodiments, the administration is accomplished via use of a pen injection device.

### BRIEF DESCRIPTION OF THE DRAWINGS

Figure 1 illustrates purity of exendin-4 in representative DMSO formulations, as determined by a SCX-HPLC methodology.

Figure 2 illustrates purity of exendin-4 in representative DMSO formulations, as determined by a RP-HPLC methodology.

Figure 3 illustrates percent of initial purity of representative DMSO formulations compared to aqueous buffer formulations held at 25 °C, as determined by SCX-HPLC methodology.

Figure 4 illustrates percent of initial purity of representative DMSO formulations compared to aqueous buffer formulations held at 40 °C, as determined by SCX-HPLC methodology.

### DETAILED DESCRIPTION OF THE INVENTION

Standard peptide and protein pharmaceutical formulations are dilute aqueous solutions. Peptide stability is usually achieved by varying one or more of the following: pH, buffer type, ionic strength, and excipients (EDTA, ascorbic acid, *etc*). For these formulations, degradation pathways requiring water (hydrolysis, deamidation, racemization) cannot be fully stabilized, and such formulations generally must be stored at sub-zero or refrigerated temperatures to protect against degradation via degradation pathways such as acid/base catalyzed hydrolysis, deamidation, racemization and oxidation. In contrast, in one aspect, incretin and incretin mimetic peptides formulated in non-aqueous solvents, such as dimethyl sulfoxide (DMSO), are shown to be chemically and physically more stable than those formulated in water. The presently claimed formulations stabilize peptide compounds at elevated temperatures (*e.g*., ranging from refrigerated temperature to about 50° C, room temperature to about 40° C, room temperature to about physiological temperature, etc.) and at high concentrations (*e.g*., 2.5% w/v, 5% w/v, 10% w/v, etc.).

In accordance with the disclosure, DMSO is an exemplary aprotic, polar solvent Without intending to be limited by theory, aprotic solvents would be expected to decrease the rate of degradation since they lack the ability to contribute protons to degradation reactions. Conversely, solvents that are more polar than water (for example, the dipole moment of water is 1.85, for DMF is 3.82, and for DMSO is 3.96) would be expected to increase the rate of degradation since they can assist in stabilizing the rate determining step. However, it has been found that the overall effect of certain aprotic, polar solvents is generally to stabilize solutions of peptides such as incretin and incretin mimetic peptides, and more specifically peptides with asparagine, glutamine, aspartic acid, glutamic acid, methionine, cysteine, tryptophan, tyrosine, histidine, lysine, and/or arginine amino acid residues.

Thus, a solution to the problem of how to achieve long-term stability and extended delivery of therapeutically active incretin and incretin mimetic peptides is disclosed, using a suitable reservoir from which the formulated peptide may be pumped or metered out at a controlled or desired rate. The reservoir may be implanted under the skin (*e.g*., as an implantable pump device) or may be external to the body and either attached or not attached (*e.g*., as a pen style injection device or external pump device). The peptide is formulated in a manner to provide stability at non-refrigerated temperatures, such as, room temperature or physiologic temperatures for the duration of therapeutic exposure, and may provide a supply of therapeutically active material for up to 2 years.

The use of aprotic, polar solvents such as DMSO to stabilize peptide formulations against both chemical and physical degradation is disclosed. It has been found that the aprotic, polar solvent may improve the overall stability of incretin and incretin mimetic peptides in a wide range of formulation conditions, including high concentrations and elevated temperatures, thus making possible the long-term storage of peptides at non-refrigerated temperatures, as well as the delivery of peptides in long-term implantable devices that would not otherwise be feasible.

The use of aprotic, polar solvents to stabilize incretin and incretin mimetic peptide formulations is disclosed, such that the peptide is released over time as a chemically unmodified form, a modified but therapeutically active form, and/or a form readily converted to a therapeutically active substance.

In one aspect is provided a co-solvent solution including DMSO with 10% water. Water at approximately 8% w/w depresses the freezing point of DMSO to just below 0°C. It has been observed that, for a DMSO solvent solution containing 10% w/w water (0.67 mole fraction DMSO), the stability of exendin-4 is enhanced.

As used herein, the following terms have the following meanings:

The term "chemical stability" means that an acceptable percentage of degradation products produced by chemical pathways such as oxidation or hydrolysis are formed. In particular, a formulation is considered chemically stable if no more than about 30%, 25%, 20%, 10%, 5%, 2% or 1% breakdown products are formed after two months at room temperature.

The term "physical stability" means that an acceptable percentage of aggregates (*e.g*., dimers, trimers and larger forms) is formed. In particular, a formulation is considered physically stable if no more that about 25%, 20%, 15%, 10%, 5%, 2% or 1% aggregates are formed after two months at room temperature.

The term "stable formulation" means that at least about 65% chemically and physically stable peptide compound remains after two months at room temperature. (or equivalent conditions at an elevated temperature). Particularly useful formulations are those which retain at least about 80% chemically and physically stable peptide under these conditions. Especially desirable stable formulations are those which do not exhibit substantial degradation after sterilizing irradiation (*e.g*., gamma, beta or electron beam).

The terms "peptide" and/or "peptide compound" mean polymers of up to about 50 amino acid residues bound together by amide (CONH) linkages. Analogs, derivatives, agonists, antagonists and pharmaceutically acceptable salts of any of these are included in these terms. The terms also include peptides and/or peptide compounds which have D-amino acids, modified, derivatized or non-naturally occurring amino acids in the D- or L-configuration and/or peptomimetic units as part of their structure.

The term "incretin or "incretin mimetic" peptide refers to a compound, for example a peptide, that directly or indirectly causes a glucose dependent increase in the amount of insulin release, such that the amount of insulin released from the pancreas is greater when plasma glucose levels are elevated as compared to when plasma glucose levels are normal. However, incretin and incretin mimetic peptides may have many additional biological functions, and the formulations and methods disclosed herein are not limited to uses solely in the context of insulin release. Specific examples of incretins include GIP and GLP-1, along with their analogs and derivatives. Examples of incretin mimetics include exendin-3 and exendin-4, along with their analogs and derivatives.

The term "high concentration" means about 2.5% w/v and up to the maximum solubility of the particular peptide.

The term "excipient" means a substance in a formulation which is added as a diluent or vehicle or to give form or consistency. By way of example, excipients include solvents such as EtOH, which are used to dissolve drugs in formulations, non-ionic surfactants such as Tween 20, which are used to facilitate solubilization of drugs in formulations, and preservatives such as benzyl alcohols or methyl or propyl parabens, which are used to prevent or inhibit microbial growth.

The term "aprotic, polar solvent" means a polar solvent which does not contain acidic hydrogen and does not act as a hydrogen bond donor. Examples of polar aprotic solvents include dimethylsulfoxide (DMSO), dimethylformamide (DMF), ethyl acetate, n-methyl pyrrolidone (NMP), dimethylacetamide (DMA), and propylene carbonate.

The term "non-aqueous protic solvent" means a non-polar solvent which contains hydrogen attached to oxygen or nitrogen so that it is able to form hydrogen bonds or donate a proton. Examples of non-aqueous protic solvents include polyethylene glycols (PEGs), propylene glycol (PG), polyvinylpyrrolidone (PVP), methoxypropylene glycol (MPEG), glycerol and glycofurol.

One aspect is drawn to pharmaceutical formulations of incretin and incretin mimetic peptide compounds as defined in the claims, in at least one aprotic, polar solvent which are stable for prolonged periods of time at elevated temperatures. Standard dilute aqueous peptide and protein formulations require manipulation of buffer type, ionic strength, pH and excipients (*e.g*., EDTA and ascorbic acid) to achieve stability. In contrast, the claimed formulations achieve stabilization of peptide compounds by the use of aprotic, polar solvents. In particular, stability of high concentrations (*e.g*., about 2.5%, w/v) of peptide compound may be provided by the formulations disclosed herein.

As mentioned above, it has unexpectedly been found that certain peptides, such as incretin and incretin mimetic peptides including GLP-1 and exendin peptides, in particular exendin peptides, formulated in the aprotic, polar solvents have improved stability when compared to formulations in water. In one aspect, stable pharmaceutical formulations are provided including incretin or incretin mimetic peptides in at least one aprotic, polar solvent that is chemically and physically stable at elevated or non-refrigerated temperatures for long periods of time. The formulations further includes one or more stabilizing excipients selected from the group consisting of: water, a sugar, and a sugar alcohol. Such formulations are stable even when high concentrations are used. Thus, these formulations are advantageous in that they may be shipped and stored at temperatures at or above non-refrigerated, room, or physiological temperature for long periods of time. They are also suitable for use in implantable delivery devices.

In certain embodiments, the peptide is solubilized in mixtures of aprotic, polar solvent(s) and the stabilizing excipients. The formulation may be a free-flowing liquid, a viscous gel-like mixture, or a freeze-dried composition. The peptide is stabilized in the formulation such that it is released as a chemically unmodified form, a modified but therapeutically active form, and/or a form readily converted to a therapeutically active substance.

In some embodiments, the incretin and incretin mimetic peptide compound may be complexed with a metal ion and the protein- or peptide-metal complex may exhibit reduced solubility in or mixtures of aprotic, polar solvent(s) and the stabilizing excipients, as compared to the dissolution of uncomplexed protein or peptide. In some embodiments, the stability of a therapeutically active peptide or protein, such as an incretin mimetic, or more particularly, exendin-3, exendin-4, or analogs or derivatives thereof, is enhanced by complexation or chelation of the peptide or protein with a metal ion, such as the zinc cation. In some embodiments, the peptide- or protein-metal complex is suspended in 0.5% water/DMSO or 10% water/DMSO, and the peptide- or protein-metal complex exhibits improved stability as compared to the dissolution of uncomplexed peptide or protein. Without wishing to be limited by theory, it is believed that complexation or chelation with the zinc cation, for example, increases the stability of a therapeutically active peptide or protein by reducing its solubility, thereby reducing susceptibility of the peptide or protein to degradation by solvolysis. Thus, subsequent suspension of the peptide- or protein-zinc complex in an aprotic polar solvent may further improve its stability as compared to dissolution of the uncomplexed protein or peptide into the solvent. In some embodiments, a dispersion of a visually observable white precipitate containing approximately 25 mg/mL exendin-4 in the form of an exendin-4-zinc complex in DMSO is obtained. In some embodiments, the dispersion of exendin-4-zinc complex in DMSO contains from about 1 mg/ml to about 100 mg/mL exendin-4. In some embodiments, the peptide-metal zinc complex is further tested for its stability at various temperatures and for varying lengths of time.

Complexation of a protein, peptide or peptide compound with a metal ion may be useful in the formulation of a beneficial agent that is delivered over a prolonged period of time. Complexation may also allow a protein, peptide or peptide compound to be formulated at a desired pH such that the formulation can be mixed or co-administered with a second beneficial agent with a reduced risk of precipitation due to pH shrift.

In one embodiment, the formulations will be in liquid form, or will be a flowable, viscous gel under conditions of use. Such formulation may exhibit a viscosity ranging from, for example, about 0.25 to 1,000,000 cP. In another embodiment, the formulations will be a lyophilized powder, which may be reconstituted prior to use.

In another aspect, the use of incretin or incretin mimetic peptides in the formulations described herein are disclosed, which peptides were lyophilized (before or after formulation) from aqueous solutions having a pH ranging from about pH 4 to about pH 7.5, about pH 4 to about pH 6, about pH4 to about pH 5, or at about a pH of 4.5, and which formulation results in increased stability. In a further aspect, the use of incretin or incretin mimetic peptides in the present formulations are disclosed, which peptides were lyophilized (before or after formulation) from aqueous solutions having a pH at or below the pI of the incretin peptide, and which formulation results in increased stability.

Incretin and incretin mimetic peptides are compounds that cause an increase in the amount of insulin released when glucose levels are normal or particularly when they are elevated. These incretin and incretin mimetic peptides have other actions beyond the initial incretin action defined by insulin secretion. For instance, they may also have actions to reduce glucagon production and delay gastric emptying. In addition, they may have actions to improve insulin sensitivity, and they may increase islet cell neogenesis - the formation of new islets.

The concept of the incretin effect developed from the observation that insulin responses to oral glucose exceeded those measured after intravenous administration of equivalent amounts of glucose. It was concluded that gut-derived factors, or incretins, influenced postprandial insulin release. Nutrient entry into the stomach and proximal gastrointestinal tract causes release of incretin hormones, which then stimulate insulin secretion. This insulinotropism, or ability to stimulate insulin secretion, can be quantified by comparing insulin or C-peptide responses to oral vs. intravenous glucose loads. In this way, it has been shown that the incretin effect is responsible for about 50% to 70% of the insulin response to oral glucose in healthy individuals.

Although many postprandial hormones have incretin-like activity, predominant incretin and incretin mimetic peptides include glucose-dependent insulinotropic polypeptide, also known as gastric inhibitory polypeptide (GIP), glucagon-like peptide-1 (GLP-1), and exendin peptides.

GIP and GLP-1 both belong to the glucagon peptide superfamily and thus share some amino acid sequence identity. GIP and GLP-1 are secreted by specialized cells in the gastrointestinal tract and have receptors located on islet cells as well as other tissues. As incretins, both are secreted from the intestine in response to ingestion of nutrients, which results in enhanced insulin secretion. The insulinotropic effect of GIP and GLP-1 is dependent on elevations in ambient glucose. Both are rapidly inactivated by the ubiquitous enzyme dipeptidyl peptidase IV (DPP-IV).

More particularly, GIP is a single 42-amino acid peptide synthesized in and secreted by specialized enteroendocrine K-cells. These cells are concentrated primarily in the duodenum and proximal jejunum, although they also can be found throughout the intestine. The main stimulant for GIP secretion is ingestion of carbohydrate- and lipid-rich meals. Following ingestion, circulating plasma GIP levels increase 10- to 20-fold. The half-life of intact GIP is estimated to be approximately 7.3 minutes in healthy subjects and 5.2 minutes in diabetic subjects.

The physiologic effects of GIP have been elucidated using GIP receptor antagonists, GIP peptide antagonists, and GIP receptor knockout mice, in addition to GIP infusion protocols. Blocking GIP binding to its receptor results in attenuated glucose-dependent insulin secretion following oral glucose load in rats and mice. Similarly, administration of GIP antagonists or GIP antisera markedly reduces the postprandial insulin release in rats. GIP receptor knockout mice demonstrate normal fasting glucose levels but mild glucose intolerance following oral glucose loads. Interestingly, they also exhibit resistance to diet-induced obesity following months of high-fat feeding. Additionally, in the leptin-deficient ob/ob mouse, the GIP receptor knockout genotype appears to decrease the extent of obesity that develops.

GIP infusion has consistently demonstrated stimulation of insulin secretion in isolated rat islets, isolated perfused rat pancreas, dogs, and humans. During stepwise euglycemic, mild hyperglycemic (54 mg/dL above basal), and moderate hyperglycemic (143 mg/dL above basal) clamps, it has been demonstrated that GIP infusion results in insulin secretion only in the presence of elevated glucose concentrations. Furthermore, it has been demonstrated that GIP is not glucagonotropic in normal humans during either euglycemic or hyperglycemic conditions. Thus, the effect of endogenously released GIP appears to be an important mechanism of postprandial insulin secretion and does not appear to play a role in the fasting state.

GIP has many non-incretin effects as well. Unlike other insulin secretagogues, GIP stimulates beta-cell proliferation and cell survival in INS-1 islet cell-line studies. Furthermore, animal studies have suggested a role for GIP in lipid metabolism by stimulating lipoprotein lipase activity, inducing fatty acid incorporation into adipose tissue and stimulating fatty acid synthesis. However, in humans, there is no clear evidence for an effect of GIP on lipid metabolism. GIP also appears to stimulate glucagon secretion from the isolated perfused rat pancreas, although human studies have not demonstrated any significant influence on glucagon secretion. Furthermore, unlike GLP-1, GIP appears to act by accelerating emptying of the stomach rather than by inhibiting gastrointestinal motility.

GLP-1, a product of the glucagon gene, is a 30/31 amino acid peptide synthesized and secreted by enteroendocrine L-cells located predominantly in the ileum and colon, although also secreted by L-cells in the duodenum and jejunum. Other incretin products of the glucagon gene include glicentin, which is biologically inactive, and oxyntomodulin, which has some insulinotropic properties. Like GIP, the GLP-1 receptor is widely expressed in pancreatic islets, the brain, heart, kidney, and the gastrointestinal tract.

There are two major forms of biologically active GLP-1 secreted following meal ingestion: GLP-1(7-37) and GLP-1 (7-36) amide, which differ by a single amino acid. The majority of the circulating active GLP-1 appears to be GLP-1 (7-36) amide, although both are equipotent and have similar biological activities. GLP-1 secretion from the distal gut is triggered by neural and endocrine signals initiated by nutrient entry into the lumen of the proximal GI tract. Circulating levels of GLP-1 increase rapidly within minutes of food ingestion and are highly correlated with the release of insulin. Like GIP, GLP-1 enhances insulin secretion in the presence of elevated glucose concentrations. DPP-IV rapidly cleaves GLP-1 to its truncated inactive metabolite. Infused GLP-1 has a shorter half-life than GIP, approximating 2 minutes in both nondiabetic and diabetic human subjects.

GLP-1 exerts many biological effects, and most of the GLP-1 actions studied in animal studies also have been demonstrated in human studies. GLP-1 is responsible for a significant part of the insulin response to oral glucose, and both animal and human studies with GLP-1 receptor antagonists suggest that GLP-1 may be essential for normal glucose tolerance. GLP-1 not only enhances insulin secretion but also suppresses the secretion of glucagon in a glucose-dependent fashion. In other words, the suppression of glucagon by GLP-1 does not occur at hypoglycemic plasma glucose concentrations but requires the presence of euglycemia or hyperglycemia. There is evidence that, like GIP, GLP-1 increases beta-cell proliferation and helps maintain populations of beta cells. GLP-1 has also been shown to slow gastric emptying in animal and human studies, resulting in slowed nutrient entry to the intestine and decreased postprandial glucose concentrations.

There is also a significant interest in the role of GLP-1 in the regulation of food intake and weight loss. In rodents, acute intracerebroventricular injection of GLP-1 or GLP-1 receptor agonists results in reduction of food intake. Furthermore, central administration of the GLP-1 receptor antagonist exendin 9-39 results in increased food intake in rats.

In summary, GLP-1: (1) stimulates glucose-dependent insulin secretion; (2) suppresses postprandial glucagon secretion; (3) reduces blood glucose after glucose loading or meal ingestion, and (4) slows gastric emptying, resulting in decreased glycemic excursion and decreased glucose-stimulated insulin secretion.

Exendins are another family of peptides implicated in insulin secretion. Exendins are found in the saliva of the Gila-monster, a lizard endogenous to Arizona, and the Mexican Beaded Lizard. Exendin-3 is present in the saliva of Heloderma horridum, and exendin-4 is present in the saliva of Heloderma suspectum (Eng, J., et al., J. Biol Chem., 265:20259-62, 1990; Eng., J., et al., J. Biol. Chem., 267:7402-05 (1992)). The exendins have some sequence similarity to several members of the glucagon-like peptide family, with the highest identity, 53%, being to GLP-1 (Goke, et al., J. Biol. Chem., 268:19650-55 (1993)).

Exendin-4 is a potent agonist at GLP-1 receptors on insulin-secreting TC1 cells, at dispersed acinar cells from guinea pig pancreas, and at parietal cells from stomach; the peptide also stimulates somatostatin release and inhibits gastrin release in isolated stomachs (Goke, et al., J. Biol. Chem., 268:19650-55 (1993); Schepp, et al., Eur. J. Pharmacol., 69:183-91 (1994); Eissele, et al., Life Sci., 55:629-34 (1994)). Exendin-3 and exendin-4 were found to be GLP-1 agonists in stimulating cAMP production in, and amylase release from, pancreatic acinar cells (Malhotra, R., et al., Relulatory Peptides, 41:149-56 (1992); Raufinan, et al., J. Biol. Chem., 267:21432-37 (1992); Singh, et al., Regul. Pept., 53:47-59 (1994)). The use of the insulinotropic activities of exendin-3 and exendin-4 for the treatment of diabetes mellitus and the prevention of hyperglycemia has been proposed (Eng, U.S. Pat. No. 5,424,286).

Truncated exendin peptides such as exendin[9-39], a carboxyamidated molecule, and: fragments 3-39 through 9-39 have been reported to be potent and selective antagonists of GLP-1 (Goke, et al., J. Biol. Chem., 268:19650-55 (1993); Raufman, J. P., et al., J. Biol. Chem., 266:2897-902 (1991); Schepp, W., et al., Eur. J. Pharm., 269:183-91 (1994); Montrose-Rafizadeh, et al., Diabetes, 45(Suppl. 2):152A (1996)). Exendin[9-39] blocks endogenous GLP-1 *in vivo,* resulting in reduced insulin secretion (Wang, et al., J. Clin. Invest., 95:417-21 (1995); D'Alessio, et al., J. Clin. Invest., 97:133-38 (1996)). The receptor apparently responsible for the insulinotropic effect of GLP-1 has been cloned from rat pancreatic islet cells (Thorens, B., Proc. Natl. Acad. Sci. USA 89:8641-8645 (1992)). Exendins and exendin[9-39] bind to the cloned GLP-1 receptor (rat pancreatic -cell GLP-1 receptor: Fehmann HC, et al., Peptides, 15 (3): 453-6 (1994); human GLP-1 receptor: Thorens B, et al., Diabetes, 42 (11): 1678-82 (1993)). In cells transfected with the cloned GLP-1 receptor, exendin-4 is an agonist, *i.e*., it increases cAMP, while exendin[9-39] is an antagonist, *i.e*., it blocks the stimulatory actions of exendin-4 and GLP-1. *Id.*

Exendin-4 is a 39 amino acid C-terminal amidated peptide found in the saliva of the Gila Monster (Heloderma horridum), with a 53% amino acid sequence identity to the GLP-1 peptide sequence. See, *e.g.,* Eng, J., et al. J. Bio. Chem., 267:11, p. 7402-7405 (1992), Young, et al., Diabetes, Vol. 48, p. 1026-1034, May, 1999. In terms of its activity, exendin-4 is a highly specific agonist for the GLP-1 receptor, and, like GLP-1, is able to stimulate insulin secretion. Therefore, like GLP-1, exendin-4 is regarded as an insulinotropic peptide.

However, unlike GIP and GLP-1, exendin-4 has a relatively long half-life in humans, because of its resistance to the dipeptidyl peptidase IV which rapidly degrades the GIP and GLP-1 sequence *in vivo.* Furthermore, it has been shown that, as compared to GLP-1, exendin-4 has a stronger capability to stimulate insulin secretion, and that a lower amount of exendin-4 may be used to obtain such stimulating activity. *See, e.g.,* U.S. Pat. No. 5,424,286, herein incorporated by reference. Therefore exendin-4 peptides or derivatives thereof (for examples of such derivatives *see, e.g.,* U.S. Pat. No. 6,528,486, and its corresponding international application WO 01/04156) have a greater potential utility for the treatment of conditions involving the dysregulation of insulin levels (*e.g*., conditions such as diabetes) than either GIP or GLP-1. Also within the scope of the invention are compositions comprising exendin agonists, exendin analogs and/or exendin agonist analogs such as those disclosed in International Patent Application Publications WO 99/25727, WO 99/25728 and WO 99/07404.

The peptide compounds useful in the formulation and methods disclosed herein can be used in the form of a salt, typically a pharmaceutically acceptable salt Useful salts are known to those of skill in the art and include salts with inorganic acids, organic acids, inorganic bases or organic bases. In one embodiment, the salts are acetate salts.

Peptides and peptide compounds which are readily soluble in the aprotic, polar solvents are especially useful, however, various excipients and solubilizing techniques known in the art may be used to enhance the solubility of a peptide of interest. One of skill in the art can easily determine which compounds will be useful on the basis of their solubility, *i.e*., the compound must be soluble in the particular aprotic, polar solvent to at least an acceptable amount. In a particular embodiment, the peptide compounds are exendin peptides, including exendin-4 and analogs thereof.

Alternatively, proteins, peptides and peptide compounds exhibiting reduced solubility are useful. In some embodiments, the stability of a therapeutically active peptide or protein, such as an incretin mimetic, or more particularly, exendin-3, exendin-4, or analogs or derivatives thereof, may be enhanced by complexation or chelation of the peptide or protein with a metal ion, such as the zinc cation. Without wishing to be limited by theory, it is believed that complexation or chelation with the zinc cation, for example, increases the stability of a therapeutically active peptide or protein by reducing its solubility, thereby reducing susceptibility of the peptide or protein to degradation by solvolysis. Thus, subsequent suspension of the peptide- or protein-zinc complex in an aprotic polar solvent is expected to further improve its stability as compared to dissolution of the uncomplexed protein or peptide into the solvent.

The proportion of incretin or incretin mimetic peptide may vary depending on the compound, the condition to be treated, the solubility of the compound, the expected dose and the duration of administration. (*See, e.g.,* The Pharmacological Basis of Therapeutics, Gilman et al., 7th ed (1985) and Pharmaceutical Sciences, Remington, 18th ed. (1990)). The concentration of peptide in high concentration formulations may range from at least about 0.05 mg/mL to the maximum solubility of the compound. In one embodiment, the range is from about 0.05 mg/mL to about 100 mg/mL, about 0.05 mg/mL to about 50 mg/mL, about 0.2 mg/mL to about 25 mg/mL, about 1.0 mg/mL to about 10.0 mg/mL, about 2.5 to about 5.0 mg/mL, etc.

Also disclosed are the *in vivo* metabolic products of the formulations described herein. Such products may result for example from the oxidation, reduction, hydrolysis, amidation, esterification and the like of the administered compound, primarily due to enzymatic processes. Accordingly, also included are compounds produced by a process comprising contacting a formulation described herein with a mammal for a period of time sufficient to yield a metabolic product thereof. Such products typically are identified by preparing a radio-labeled (*e.g*. C¹⁴ or H³) formulation described herein, administering it in a detectable dose (*e.g*., greater than about 0.5 mg/kg) to a mammal such as rat, mouse, guinea pig, monkey, or to man, allowing sufficient time for metabolism to occur (typically about 30 seconds to 30 hours), and isolating its conversion products from urine, blood or other biological samples. These products are easily isolated since they are labeled (others are isolated by the use of antibodies capable of binding epitopes surviving in the metabolite). The metabolite structures are determined in conventional fashion, *e.g*., by MS or NMR analysis. In general, analysis of metabolites may be done in the same way as conventional drug metabolism studies well-known to those skilled in the art. The conversion products, so long as they are not otherwise found *in vivo*, are useful in diagnostic assays for therapeutic dosing of the formulations described herein, even if they possess no biological activity of their own.

Generally, the stable formulations described herein may be prepared by simply dissolving the desired amount, which may be a therapeutically effective amount, of the desired peptide compound in the selected aprotic, polar solvent. Exemplary aprotic, polar solvents include dimethylsulfoxide (DMSO), dimethylformamide (DMF), ethyl acetate, n-methyl pyrrolidone (NMP), dimethylacetamide (DMA), propylene carbonate, and mixtures thereof.

The aprotic, polar solvent may optionally include minor amounts of water in desired quantities. Increasing the water contained in the peptide formulations may generally increase peptide degradation. However, the stabilization effect of the formulations may nevertheless be sufficient to result in acceptable long-term stability.

In one embodiment, the aprotic, polar solvent has a freezing point at or below about 0°C, so as to avoid freezing during storage. In this regard, without intending to be limited by theory, it is believed that peptide stability is promoted by avoiding phase changes. As such, in one aspect, the formulations may exhibit a freezing point below about 0°C. Such a freezing point may be an inherent property of the aprotic, polar solvent, or alternative, co-solvent systems may be used to obtain the desired freezing point.

Any pharmaceutically acceptable components which function to enhance the solubility, physical stability, and/or chemical stability of the incretin or incretin mimetic peptide in the formulations of the invention may be useful in the context of the formulations described herein. The pharmaceutical formulations described herein include one or more stabilizing excipient, and each excipient may have one or more stabilizing functions.

In one aspect, the stabilizing excipient may function to stabilize the physical nature of the peptide. Suitable stabilizing excipients capable of stabilizing the incretin or incretin mimetic peptide water, include sugars, sugar alcohols, or mixtures thereof.

In some embodiments, the stabilizing excipient may function to stabilize the peptide against chemical degradation, *e.g*., by reducing or preventing the formation of cyclic imide or other degradation products of asparagine and glutamine amino acid residues.

A reduction in chemical degradation may be observed as, for example, a reduction of about 2%, about 5%, about 6%, about 7%, about 8%, about 9%, about 10%, about 15%, about 20%, about 25%, about 30%, about 35%, about 40%, about 50%, about 60%, about 70%, about 80%, or about 90% in the rate of degradation of the formulation comprising an aprotic, polar solvent and/or at least one stabilizing excipient, as compared to the rate of degradation of the formulation not containing an aprotic, polar solvent and/or at least one stabilizing excipient

In some embodiments, a formulation is considered to exhibit a reduction in chemical breakdown products if a lesser percentage of breakdown products is observed after one month at 25°C in the formulation comprising an aprotic, polar solvent and/or at least one stabilizing excipient, as compared to the formulation not containing an aprotic, polar solvent and/or at least one stabilizing excipient. In some embodiments, a formulation is considered to exhibit a reduction in chemical breakdown products if a lesser percentage of breakdown products is observed after two months at 25°C in the formulation comprising an aprotic, polar solvent and at least one stabilizing excipient, as compared to the formulation not containing an aprotic, polar solvent and at least one stabilizing excipient. In some embodiments, a formulation is considered to exhibit a reduction in chemical breakdown products if a lesser percentage of breakdown products is observed after three months at 25°C in the formulation comprising an aprotic, polar solvent and at least one stabilizing excipient, as compared to the formulation not containing an aprotic, polar solvent and at least one stabilizing excipient. In some embodiments, a formulation is considered to exhibit a reduction in chemical breakdown products if a lesser percentage of breakdown products is observed after six months at 25°C in the formulation comprising an aprotic, polar solvent and at least one stabilizing excipient, as compared to the formulation not containing an aprotic, polar solvent and at least one stabilizing excipient. In some embodiments, a formulation is considered to exhibit a reduction in chemical breakdown products if a lesser percentage of breakdown products is observed after one month at 37°C in the formulation comprising an aprotic, polar solvent and at least one stabilizing excipient, as compared to the formulation not containing an aprotic, polar solvent and at least one stabilizing excipient. In some embodiments, a formulation is considered to exhibit a reduction in chemical breakdown products if a lesser percentage of breakdown products is observed after two months at 37°C in the formulation comprising an aprotic, polar solvent and at least one stabilizing excipient, as compared to the formulation not containing an aprotic, polar solvent and at least one stabilizing excipient In some embodiments, a formulation is considered to exhibit a reduction in chemical breakdown products if a lesser percentage of breakdown products is observed after three months at 37°C in the formulation comprising an aprotic, polar solvent and at least one stabilizing excipient, as compared to the formulation not containing an aprotic, polar solvent and at least one stabilizing excipient. In some embodiments, a formulation is considered to exhibit a reduction in chemical breakdown products if a lesser percentage of breakdown products is observed after six months at 37°C in the formulation comprising an aprotic, polar solvent and at least one stabilizing excipient, as compared to the formulation not containing an aprotic, polar solvent and at least one stabilizing excipient.

In yet another aspect, the stabilizing excipient may function to depress the freezing point of the aprotic, polar solvent to 0°C or below. Freezing points below 0°C are believed to stabilize the formulation by preventing phase changes at likely conditions of preparation and storage. In this regard, without intending to be limited by theory, it is believed that physical stability of the peptide is maintained through minimizing phase changes. Suitable excipients in this context are water, salts, sugars, sugar alcohols, and mixtures thereof.

In yet another aspect, the stabilizing excipient may function to increase the viscosity of the formulation to within the range of 0.25 to 1,000,000 cP. Examples of suitable stabilizing excipients in this context include thermo-responsive polymers which increase the viscosity of the formulation, but do not gel at the conditions of use.

In one embodiment, the stabilizing excipient may be a non-aqueous protic solvent. Examples of suitable non-aqueous protic solvents include polyethylene glycols (PEGs), propylene glycol (PG), polyvinylpyrrolidone (PVP), methoxypropylene glycol (MPEG), glycerol and glycofurol.

In another embodiment, the stabilizing excipient may be an aqueous buffer, an antioxidant, a chelator, a surfactant, or any other pharmaceutically acceptable additive that enhance solubility or stability of the peptide. Examples of suitable buffers include acetate, citrate, phosphate, tartrate, and glutamate buffers. Examples of suitable antioxidants include ascorbic acid, cysteine, methionine, monothioglycerol, sodium thiosulphate, sulfites, BHT, BHA, ascorbyl palmitate, propyl gallate, Vitamin E, or mixtures thereof. Examples of suitable chelators include EDTA, glycerin, tartaric acid and salts thereof, citric acid and salts thereof, or mixtures of any of the preceding.

In another aspect, uses of the formulations described herein are provided. The uses generally comprise a formulation described herein for administration to a subject in need thereof. The formulations can be used in any therapeutic or prophylactic context in which the incretin or incretin mimetic peptide may be useful. By way of non-limiting example, the uses may include treatment or prevention of diabetes mellitus (including Type 1, Type 2, and gestational), glucose intolerance, obesity, dyslipidemia, myocardial infarction, on any other known use of incretin or incretin mimetic peptides.

In accordance with the uses disclosed herein, a pharmaceutical formulation may be for administration in any manner known in the art which renders the incretin or incretin mimetic peptide biologically available to the subject or sample in effective amounts. For example, the formulation may for administration to a subject *via* any central or peripheral route known in the art including, but not limited to: oral, parenteral, transdermal, transmucosal, or pulmonary routes. In one embodiment, parenteral administration is used. Specific exemplary routes of administration include oral, ocular, rectal, buccal, topical, nasal, ophthalmic, subcutaneous, intramuscular, intraveneous, intracerebral, transdermal, and pulmonary. Determination of the appropriate administration method is usually made upon consideration of the condition (*e.g*., disease or disorder) to be treated, the stage of the condition (*e.g*., disease or disorder), the comfort of the subject, and other factors known to those of skill in the art.

Administration may be intermittent or continuous, both on an acute and/or chronic basis. Continuous administration may be achieved using an implantable or attachable pump controlled delivery device, such as described in U.S. Patent Nos. 5,728,396; 5,985,305; 6,156,331; 6,261,584, and 6,395,292. However, any implanted controlled delivery device known in the art may be used. Alternatively, pen style injection devices known in the art may be used in conjunction with the formulations and methods described herein.

In one embodiment, administration can be a prophylactic treatment, beginning concurrently with the diagnosis or observation of condition(s) (*e.g*., lifestyle, genetic history, surgery, *etc*.) which places a subject at risk of developing a specific disease or disorder. In the alternative, administration can occur subsequent to occurrence of symptoms associated with a specific disease or disorder.

The term "effective amount" refers to an amount of a pharmaceutical agent used to treat, ameliorate, prevent, or eliminate the identified condition (*e.g*., disease or disorder), or to exhibit a detectable therapeutic or preventative effect. The effect can be detected by, for example, chemical markers, antigen levels, or time to a measurable event, such as morbidity or mortality. The precise effective amount for a subject will depend upon the subject's body weight, size, and health; the nature and extent of the condition; and the therapeutic or combination of therapeutics selected for administration. Effective amounts for a given situation can be determined by routine experimentation that is within the skill and judgment of the clinician.

For any peptide, the effective amount can be estimated initially either in cell culture assays, *e.g*., in animal models, such as rat or mouse models. An animal model may also be used to determine the appropriate concentration range and route of administration. Such information can then be used to determine useful doses and routes for administration in humans.

Efficacy and toxicity may be determined by standard pharmaceutical procedures in cell cultures or experimental animals, *e.g*., ED₅₀ (the dose therapeutically effective in 50% of the population) and LD₅₀ (the dose lethal to 50% of the population). The dose ratio between therapeutic and toxic effects is the therapeutic index, and it can be expressed as the ratio, ED₅₀/LD₅₀. Pharmaceutical compositions that exhibit large therapeutic indices are preferred. The data obtained from cell culture assays and animal studies may be used in formulating a range of dosage for human use. The dosage contained in such compositions is typically within a range of circulating concentrations that include an ED₅₀ with little or no toxicity. The dosage may vary within this range depending upon the dosage form employed, sensitivity of the patient, and the route of administration.

More specifically, the concentration-biological effect relationships observed with regard to the incretin or incretin mimetic peptides employed in the methods disclosed herein indicate that a target dose will be in the range of about 1 µg/day to about 1 g/day, or about 10 µg/day to about 10 mg/day, or about 10 µg/day to about 250 µg/day, about 10 µg/day to about 50 µg/day, or about 20 µg/day, in single, divided, or continuous doses for a patient weighing between about 50 to about 100 kg. Dosages may be adjusted accordingly for patients above or below the stated weight range. The exact dosage will be determined by the practitioner, in light of factors related to the subject that requires treatment.

Disclosed herein is the identification of a subject in need of treatment. Any effective criteria may be used to determine that a subject may benefit from administration of an incretin or incretin mimetic peptide. Methods for the diagnosis of heart disease, obesity, dyslipidemia, and diabetes, for example, as well as procedures for the identification of individuals at risk for development of these conditions, are well known to those in the art. Such procedures may include clinical tests, physical examination, personal interviews and assessment of family history.

To assist in understanding the present invention, the following Examples are included. The experiments described herein should not, of course, be construed as specifically limiting the invention.

### EXAMPLES

The present invention is described in more detail with reference to the following non-limiting examples, which are offered to more fully illustrate the invention, but are not to be construed as limiting the scope thereof.

### Example 1:

The stability of exendin-4 in DMSO and DMSO with 0.5% water added may be evaluated as follows. The evaluation may be based on the stability of exendin-4 samples stored at 5, 25, and 40°C for up to 6 months. Further, the stability of exendin-4 in DMSO, as compared to aqueous buffers at a pH of 4.5 may be evaluated.

Three HPLC methods may be used to analyze the samples: size exclusion HPLC (SEC-HPLC) to determine potency (mg/ml) and two methods to evaluate purity (%), a strong cation exchange (SCX) method and a reversed-phase (RP) method. The methods may be adapted as necessary to achieve appropriate sample analysis. Additionally, the water content of the samples may be evaluated using a suitable Karl Fischer analytical procedure.

For example, SEC-HPLC can be used to measure the potency of an exendin-4 solution by external standard assay, based on total peptide content of the exendin-containing solution at 214 nm, as compared to qualified reference standard solutions. The identity, potency and label strength of exendin-4 can be established by comparison of the retention times of the exendin-4 peaks in the sample and reference standard solutions.

For a six-month delivery period, approximately 3600 µg of exendin-4 may be desirable based on a 20 µg/day dose. Assuming a representative delivery reservoir of approximately 150 µL, a concentration of about 25 mg/mL may be desirable. Further, proteins and peptides are commonly lyophilized and often include some residual moisture. As such, the effect of water on peptide stability may be investigated, and samples may be prepared in neat DMSO and in DMSO with 0.5% water added.

This concentration of water will depress the 18.6°C freezing point of DMSO to only about 17.5°C. Because of the low molar concentration, the peptide is expected to further depress the freezing point only slightly. Samples may be prepared using exendin-4 desiccated by storing in a nitrogen-filled desiccator, over phosphorus pentoxide, for at least 24 hours. A nitrogen-filled glove bag may be used to prepare bulk solution and individual samples. The desiccator, equipment, and supplies may be placed in the glove bag. The glove bag may be flushed with nitrogen and sealed. Samples may be prepared by adding approximately 1250 mg exendin-4 to a 50 mL volumetric flask. DMSO (sealed container) may be added to achieve the final volume. Approximately one-half of the solution may then be transferred to a 25 mL volumetric flask, 125 µL water may be added, and the resulting solution mixed to obtain a final solution of 0.5% w/v water. Samples may then be portioned into separate 2-mL vials, capped, and crimp sealed. The samples may be stored, e.g., at 5°C, 25°C, and 40°C in cardboard boxes to provide protection from light. Samples may then be tested at desired intervals for purity and potency.

Results from representative samples of exendin-4 prepared in a manner similar to that described above are provided in the tables below. More particularly, 25 mg/mL samples of exendin-4 in neat DMSO and DMSO/0.5% water are prepared in N₂ atmosphere and held at 5, 25, and 40°C for 6 months (abbreviated as "mos.").

| **Method** | **Temp (°C)** | **System** | **Potency (mg/mL)** | | | | |
|---|---|---|---|---|---|---|---|
| SEC-HPLC | | | 0 mos. | 1 mos. | 2 mos. | 3 mos. | 6 mos. |
| Potency (mg/mL) | 5 | neat DMSO | | | | | 24.5 |
| | 5 | 0.5% water/DMSO | | | | | 24.4 |
| | | | | | | | |
| | 25 | neat/DMSO | 23.4 | 24.8 | 24.5 | 24.4 | 24.3 |
| | 25 | 0.5% water/DMSO | 23.4 | 24.9 | 24.5 | 24.4 | 24.2 |
| | | | | | | | |
| | 40 | neat DMSO | | 24.8 | 24.6 | 24.7 | 23.6 |
| | 40 | 0.5% water/DMSO | | 24.9 | 24.7 | 24.4 | 23.1 |

| **Method (°C)** | **Temp** | **System** | **%Purity** | | | |
|---|---|---|---|---|---|---|
| RP-HPLC | | | 1 mos. | 2 mos. | 3 mos. | 6 mos. |
| %Purity | 5 | neat DMSO | | | | 99.7 |
| | 5 | 0.5% water/DMSO | | | | 99.6 |
| | 25 | neat/DMSO | 99.7 | 99.1 | 98.4 | 97.0 |
| | 25 | 0.5% water/DMSO | 99.5 | 99.2 | 98.3 | 96.6 |
| | 40 | neat DMSO | 96.5 | 90.2 | 83.7 | 71.1 |
| | 40 | 0.5% water/DMSO | 96.3 | 90.3 | 83.8 | 71.7 |

| **Method** | **Temp (°C)** | **System** | **%Purity** | | | |
|---|---|---|---|---|---|---|
| SCX-HPLC | | | 1 mos. | 2 mos. | 3 mos. | 6 mos. |
| %Purity | 5 | neat DMSO | | | | 99.5 |
| | 5 | 0.5% water/DMSO | | | | 99.4 |
| | 25 | neat/DMSO | 99.0 | 98.4 | 96.7 | 95.8 |
| | 25 | 0.5% water/DMSO | 99.2 | 98.3 | 97.9 | 95.8 |
| | 40 | neat DMSO | 95.8 | 90.2 | 85.0 | 72.2 |
| | 40 | 0.5% water/DMSO | 96.0 | 90.7 | 85.7 | 72.5 |

| **Method** | **Temp (°C)** | **System** | **%Purity** | | | | |
|---|---|---|---|---|---|---|---|
| SCX-HPLC | | | 0.25 mos. | 0.5 mos. | 1 mos. | 2 mos. | 3 mos. |
| %Purity | 25 | Aqueous Buffer (pH 4.5) | 99.2 | 98.5 | 97.1 | 94.8 | 90.4 |
| | 40 | Aqueous Buffer (pH 4.5) | 92.2 | 87.9 | | | |

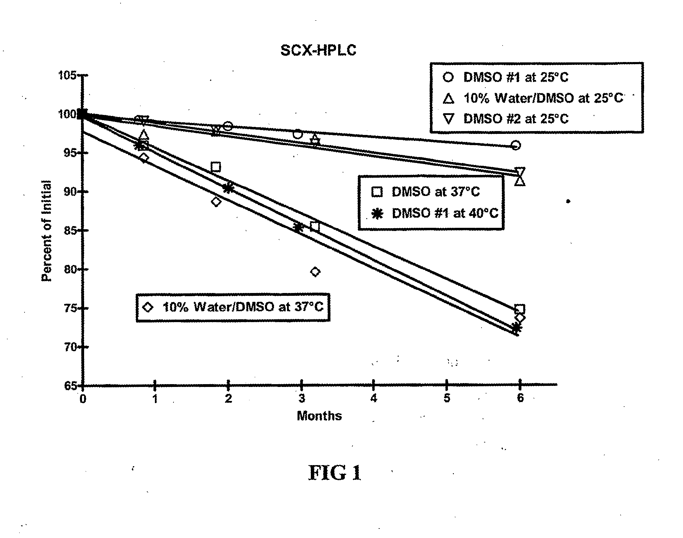

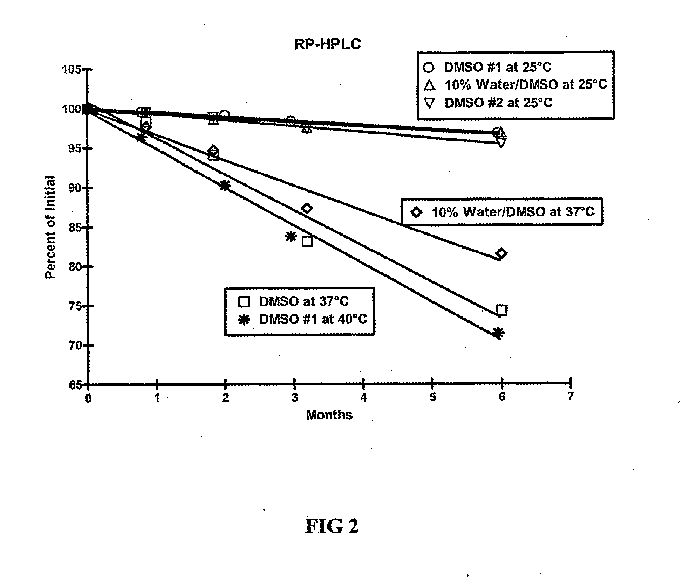

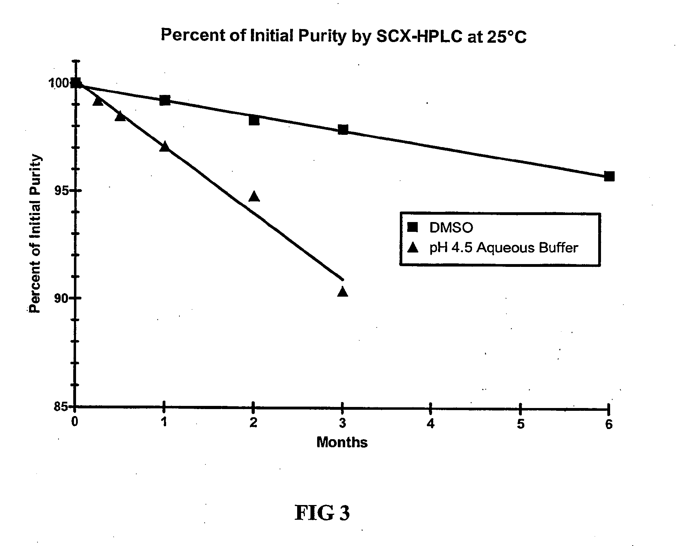

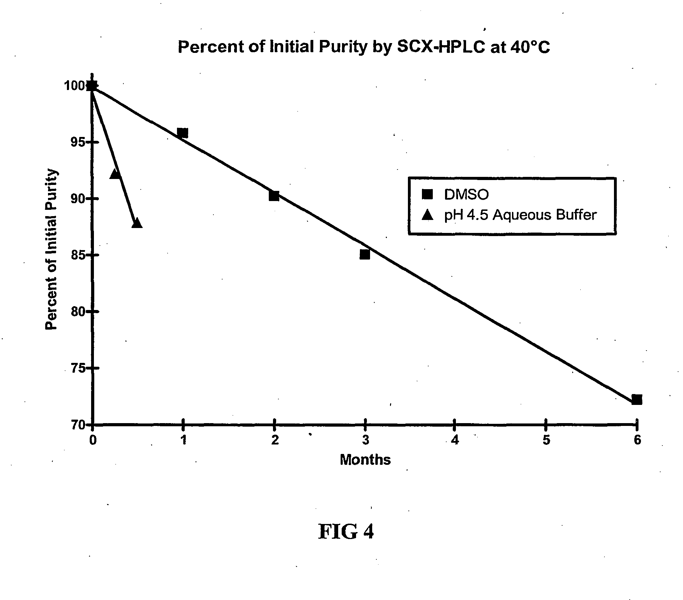

Exendin-4 may be formulated in DMSO and DMSO spiked with 0.5% water. Briefly, as demonstrated above, there is no substantial difference due to the presence of 0.5% water that might be introduced from residual moisture of a lyophilized peptide. Exendin-4 purity is reduced about 28% from initial values after 6 months at 40°C. At 25°C, the purity is decreased about 3 to 4% over the same time period. (See Figures 1 and 2). However, at 5°C the purity remains within 0.4 to 0.6% of initial purity values. Essentially no changes in potency are observed. By comparison to aqueous product stability (Figures 3 and 4), it is apparent that exendin-4 stability is improved in the aprotic, polar solvent DMSO.

### Example 2: Exendin-4 Stability in Aprotic, Polar Solvent Systems

As demonstrated in Example 1, DMSO provides improved stability of exendin-4. The stability of exendin-4 in other aprotic, polar solvents and in DMSO-based co-solvent systems may also be evaluated. The evaluation may be based on the stability of exendin-4 samples stored at 5, 25, and 37°C for up to 6 months. In addition to dimethyl sulfoxide (DMSO), solvents for evaluation include water, dimethyl acetamide (DMA), dimethyl formamide (DMF), N-methylpyrrolidorie (NMP), propylene carbonate, and ethyl acetate.

Three HPLC methods may be used to analyze the samples: size exclusion HPLC (SEC-HPLC) to determine potency (mg/ml) and two methods to evaluate purity (%), a strong cation exchange (SCX) method and a reversed-phase (RP) method. The methods may be adapted as necessary to achieve appropriate sample analysis. Additionally, the water content of the samples may be evaluated using a suitable Karl Fischer analytical procedure.

For example, SEC-HPLC can be used to measure the potency of an exendin-4 solution by external standard assay, based on total peptide content of the exendin-containing solution at 214 nm, as compared to qualified reference standard solutions. The identity, potency and label strength of exendin-4 can be established by comparison of the retention times of the exendin-4 peaks in the sample and reference standard solutions.

Nonaqueous Solvents: The stability of exendin-4 in aprotic, polar solvents with freezing points lower than 0°C may be evaluated. Representative solvents that meet these criteria are DMA, DMF, NMP, propylene carbonate, and ethyl acetate.

Co-solvent Systems with Water: Water at approximately 8% w/w depresses the freezing point of DMSO to just below 0°C. To provide additional protection against freezing, a 10% w/w water solution may be evaluated. Addition of water to the system provides the possibility of hydrolysis reactions with the peptide. However, there is a strong interaction between DMSO and water molecules that may mitigate the hydrolysis reactions. In fact, for 10% w/w water (0.67 mole fraction DMSO), it has been shown that the mixture is characterized by 1:1 DMSO:water complexes. It is only where the mole fraction of water exceeds 0.6 that pure water molecules are prevalent. Additionally, the hydrolysis reactions are increased at pH extremes, indicating catalysis is by hydronium and hydroxyl ions. Ionization of many compounds, including water, is suppressed in DMSO and the pKa of water is shifted from 15.75 for pure water to 32 in DMSO solution. This corresponds to a reduction in hydronium and hydroxyl ions in neutral solution of greater than 1 x 10⁸ M. Thus, it is desired to evaluate exendin-4 stability in this system.

Co-solvent Systems with Aprotic, Polar Solvents: Other solvents can also be used to depress the freezing point of DMSO. Thus, binary solvent systems may be prepared using DMSO and DMA, DMF, NMP, propylene carbonate, or ethyl acetate. These mixtures may be designed to take advantage of an improved solubility and/or stability provided by a DMSO-rich mixture. Appropriate amounts of the non-aqueous solvent may first be determined. Next the solubility of exendin-4 may be evaluated by visual inspection in the co-solvent mixture. Systems providing sufficient exendin-4 solubility and not freezing in the refrigerator may be chosen for stability analysis.

DMSO: An 10 mg/mL exendin-4 solution in DMSO may be prepared for use as a control.

Many of the nonaqueous solvents of interest are very hygroscopic and can absorb water when exposed to the atmosphere. Furthermore, the samples may be portioned into partially filled containers. Thus, a nitrogen-filled glove bag or box may be used to prepare bulk solution and individual samples. Samples may be placed in the glove bag. The glove bag may be flushed with nitrogen and sealed. Samples may be prepared by adding approximately 110 mg exendin-4 to a glass vial. Non-aqueous solvent or co-solvent mixture may then be added to achieve the final concentration of about 10 mg/mL. Samples (approximately 0.5 mL) may be portioned into separate 2 mL vials, capped, and crimp sealed. The samples may be stored at 5°C, 25°C, and 37°C in cardboard boxes to provide protection from light. Samples may then be tested at one month, two months, three months and six months, as desired.

Representative samples of exendin-4 are prepared in a manner similar to that described above. More particularly, the following samples are prepared:

| **Formulation No.** | **Solvent System** | **Mixture % w/w solvent in DMSO** |
|---|---|---|
| 1 | Ethyl Acetate/DMSO | 33% w/w |
| 2 | Propylene carbonate/DMSO | 30% w/w |
| 3 | N-methylpyrrolidone/DMSO | 26% w/w |
| 4 | Dimethyl formamide/DMSO | 25% w/w |
| 5 | Dimethyl acetamide/DMSO | 30% w/w |
| 6 | Water/DMSO | 10% w/w |
| 7 | N-methylpyrrolidone | (neat) |
| 8 | Dimethyl formamide | (neat) |
| 9 | Dimethyl acetamide | (neat) |
| 10 | DMSO | (neat) |

Results from representative samples are provided in the tables below.

| | **PERCENT OF INITIAL VALUE** | | | | | | | | |
|---|---|---|---|---|---|---|---|---|---|
| | **MONTHS** | | | | | | | | |
| **SCX-Purity** | | **37°C** | | | | **25°C** | | | |
| **Form.** | **Initial** | **1** | **2 No.** | **3** | **6** | **1** | **2** | **3** | **6** |
| 1 | 98.8 | 64.0 | 32.9 | | | 85.1 | 71.3 | | |
| 2 | 97.1 | 16.4 | | | | 51.3 | | | |
| 3 | 97.6 | 88.0 | 77.7 | 62.0 | 52.1 | 95.8 | 90.0 | 83.3 | 78.1 |
| 4 | 98.6 | 19.8 | | | | 60.5 | | | |
| 5 | 99.0 | 86.4 | 72.2 | 53.5 | 40.9 | 94.0 | 89.6 | 86.9 | 77.6 |
| 6 | 99.1 | 94.3 | 88.7 | 79.6 | 73.6 | 97.3 | 97.8 | 96.8 | 91.3 |
| 7 | 81.4 | 30.7 | | | | 52.1 | | | |
| 8 | 92.9 | 5.6 | | | | 28.1 | | | |
| 9 | 98.3 | 65.7 | 28.7 | | | 87.3 | 76.7 | | |
| 10 | 99.1 | 95.9 | 93.1 | 85.4 | 74.7 | 99.0 | 97.8 | 96.1 | 92.3 |

| | **PERCENT OF INITIAL VALUE** | | | | | | | | |
|---|---|---|---|---|---|---|---|---|---|
| | **MONTHS** | | | | | | | | |
| **RP-Purity** | | **37°C** | | | | **25°C** | | | |
| **Form. No.** | **Initial** | **1** | **2** | **3** | **6** | **1** | **2** | **3** | **6** |
| 1 | 97.0 | 62.2 | 32.8 | | | 86.8 | 73.2 | | |
| 2 | 95.4 | 14.2 | | | | 49.6 | | | |
| 3 | 96.2 | 90.4 | 77.2 | 62.5 | 47.3 | 95.5 | 91.3 | 83.7 | 76.5 |
| 4 | 96.5 | 20.1 | | | | 64.1 | | | |
| 5 | 97.5 | 88.3 | 74.1 | 50.8 | 39.0 | 96.2 | 92.5 | 86.8 | 78.7 |
| 6 | 97.7 | 97.7 | 94.7 | 87.3 | 81.5 | 99.5 | 98.7 | 97.6 | 96.9 |
| 7 | 86.6 | 32.5 | | | | 59.9 | | | |
| 8 | 91.8 | 0.4 | | | | 26.5 | | | |
| 9 | 96.8 | 68.4 | 28.6 | | | 89.4 | 80.7 | | |
| 10 | 97.4 | 98.0 | 94.1 | 83.1 | 74.3 | 99.5 | 98.9 | 97.4 | 95.5 |

| | **PERCENT OF INITIAL VALUE** | | | | | | | | |
|---|---|---|---|---|---|---|---|---|---|
| | **MONTHS** | | | | | | | | |
| **SEC-Potency** | | **37°C** | | | | **25°C** | | | |
| **Form. No.** | **Initial** | **1** | **2** | **3** | **6** | **1** | **2** | **3** | **6** |
| 1 | 9.05 | 107.0 | 107.5 | | | 103.3 | 102.5 | | |
| 2 | 8.24 | 99.0 | | | | 98.4 | | | |
| 3 | 8.52 | 100.1 | 103.0 | 96.3 | 91.6 | 99.9 | 101.2 | 98.6 | 97.7 |
| 4 | 8.80 | 103.2 | | | | 99.5 | | | |
| 5 | 9.06 | 100.8 | 100.8 | 99.3 | 101.5 | 100.5 | 100.0 | 100.2 | 98.9 |
| 6 | 9.48 | 101.6 | 102.1 | 102.2 | 99.2 | 98.5 | 100.7 | 100.9 | 99.6 |
| 7 | 9.57 | 98.2 | | | | 98.7 | | | |
| 8 | 9.27 | 103.3 | | | | 102.2 | | | |
| 9 | 9.02 | 102.2 | 100.5 | | | 102.2 | 103.7 | | |
| 10 | 9.42 | 102.7 | 102.1 | 102.7 | 98.9 | 100.9 | 101.9 | 101.5 | 101.9 |

### Example 3: Increased Stability of Peptide-Zinc Complexes in Aprotic, Polar Solvent Systems

As one means of increasing the stability of a therapeutically active incretin or incretin mimetic peptide compound, such as exendin-3, exendin-4, or analogs or derivatives thereof, the peptide may be complexed with a metal ion, such as the zinc cation. Without wishing to be limited by theory, it is believed that complexation or chelation with the zinc cation, for example, increases the stability of a therapeutically active incretin or incretin mimetic peptide by reducing its solubility, thereby reducing susceptibility of the peptide to degradation by solvolysis. Thus, subsequent suspension of the peptide-zinc complex in an aprotic polar solvent is expected to further improve its stability as compared to dissolution of the uncomplexed peptide into the solvent. The stability of an exendin-4-zinc complex in suspension with DMSO, DMSO with 0.5% water added, in other aprotic, polar solvents (for example, water, DMA, DMF, NMP, propylene carbonate or ethyl acetate), in DMSO-based co-solvent systems as described above, or in aprotic non-polar solvents (for example, silicone oil or dimethicone) may be evaluated. The evaluation may be based on the stability of exendin-4-zinc samples stored at 5, 25, 37 and/or 40°C for up to 6 months. Further, the stability of exendin-4 in DMSO as compared to aqueous buffers at a pH ranging from about pH 4.0 to about pH 7.5 may be evaluated.

Three HPLC methods may be used to analyze the samples: size exclusion HPLC (SEC-HPLC) to determine potency (mg/ml) and two methods to evaluate purity (%), a strong cation exchange (SCX) method and a reversed-phase (RP) method. The methods may be adapted as necessary to achieve appropriate sample analysis. Additionally, the water content of the samples may be evaluated using a suitable Karl Fischer analytical procedure.

For example, SEC-HPLC can be used to measure the potency of an exendin-4-zinc solution by external standard assay, based on total peptide content of the solution containing exendin-4-zinc at 214 nm, as compared to qualified reference standard solutions. The identity, potency and label strength of exendin-4-zinc are established by comparison of the retention times of the exendin-4-zinc peaks in the sample and reference standard solutions.

Complex formation: Exendin-4 is mixed with zinc and the exendin-4-zinc complex is found to precipitate at neutral pH. In a 20 mL beaker, a clear solution containing approximately 10.7 mg/mL exendin-4 is made by dissolving 0.1074 grams of exendin-4 and 1.16158 grams of zinc acetate dihydrate in 10 mL deionized water. The starting pH of this solution is 5.73. When the pH of the solution is adjusted to 7.00 by dropwise addition of a 45% w/w potassium hydroxide solution, the solution becomes cloudy and a white precipitate (exendin-4-zinc complex) is observed. 5 mL of the cloudy suspension of exendin-4-zinc complex is then transferred to a 15 mL centrifuge tube and centrifuged at 4000 rpm for 5 minutes, and the supernatant removed.

Dispersion in DMSO: Peptide-metal complexes may be suspended in aprotic polar solvents, such as DMSO, 0.5% water/DMSO or 10% water/DMSO to form a suspension in which the peptide-metal complex may exhibit improved stability. For example, to the exendin-4-zinc complex precipitated as described above, 2 mL DMSO is added, and contents mixed by inversion. A dispersion of a visually observable white precipitate containing approximately 25 mg/mL exendin-4 in DMSO is obtained, indicating that the exendin-4-zinc complex does not dissolve in DMSO. This dispersion can be further tested for its stability at various temperatures and for varying lengths of time.

## Claims

1. A stable pharmaceutical formulation comprising an incretin or incretin mimetic peptide and at least one aprotic, polar solvent, and at least one stabilizing excipient selected from the group consisting of: water, a sugar, and a sugar alcohol.

2. The stable pharmaceutical formulation of claim 1, wherein the incretin or incretin mimetic peptide is selected from the group consisting of: a glucagon-like-peptide 1 (GLP-1) peptide and an exendin peptide or an analog thereof.

3. The stable pharmaceutical formulation of claim 2, wherein the exendin peptide is exendin-4 or an analog thereof.

4. The stable pharmaceutical formulation of claim 1, wherein the stabilizing excipient is water.

5. The stable pharmaceutical formulation of any one of claims 1-4, wherein the at least one aprotic, polar solvent is selected from the group consisting of: dimethylsulfoxide (DMSO), dimethylformamide (DMF), ethyl acetate, n-methyl pyrrolidone (NMP), dimethylacetamide (DMA), propylene carbonate, and mixtures thereof.

6. The stable pharmaceutical formulation of claim 5, wherein the at least one aprotic, polar solvent is DMSO.

7. The stable pharmaceutical formulation of claim 1, wherein the at least one stabilizing excipient is capable of depressing the freezing point of the aprotic, polar solvent to about 0°C or below.

8. The stable pharmaceutical formulation of claim 7, wherein the aprotic, polar solvent is DMSO, wherein the at least one stabilizing excipient capable of depressing the freezing point of the aprotic, polar solvent is water, and the water and DMSO form a co-solvent comprising 10% w/w water (0.67 mole fraction DMSO).

9. The stable pharmaceutical formulation of any one of claims 1-8, wherein the at least one stabilizing excipient is capable of stabilizing the conformation of the incretin or incretin mimetic peptide.

10. The stable pharmaceutical formulation of any one of claims 1-9, wherein the incretin peptide comprises one or more amino acid residues selected from the group consisting of: asparagine, glutamine, aspartic acid, glutamic acid, methionine, cysteine, tryptophan, tyrosine, histidine, lysine, and arginine, and the aprotic, polar solvent and/or the at least one stabilizing excipient stabilizes the amino acid residue from degradation.

11. The stable pharmaceutical formulation of claim 10, wherein the amino acid residue is selected from the group consisting of: asparagine and glutamine, and the aprotic, polar solvent and/or the at least one stabilizing excipient stabilizes the amino acid residue against degradation by reducing or preventing the formation of cyclic imide or other degradation products of asparagine and glutamine amino acid residues.

12. The stable pharmaceutical formulation of any one of claims 1-11, further comprising at least one non-aqueous protic solvent.

13. The stable pharmaceutical formulation of claim 12, wherein the non-aqueous protic solvent is selected from the group consisting of: polyethylene glycol (PEG), propylene glycol (PG), polyvinylpyrrolidone (PVP), methoxypropylene glycol (MPEG), glycerol, glycofurol, and mixtures thereof.

14. The stable pharmaceutical formulation of any one of claims 1-13, further comprising a buffer.

15. The stable pharmaceutical formulation of claim 14, wherein the buffer is an acetate buffer.

16. The stable pharmaceutical formulation of any one of claims 1-15, further comprising an antioxidant.

17. The stable pharmaceutical formulation of claim 16, wherein the antioxidant is selected from at least one of the group consisting of: ascorbic acid, cysteine, methionine, monothioglycerol, sodium thiosulphate, sulfites, BHT, BHA, ascorbyl palmitate, propyl gallate, and Vitamin E.

18. The stable pharmaceutical formulation of any one of claims 1-17, further comprising a chelator.

19. The stable pharmaceutical formulation of claim 18, wherein the chelator.is selected from at least one of the group consisting of EDTA, tartaric acid and salts thereof, glycerin, and citric acid and salts thereof.

20. The stable pharmaceutical formulation of any one of claims 1-19, further comprising a non-aqueous solvent.

21. The stable pharmaceutical formulation of claim 20, wherein the nonaqueous solvent is at least one selected from the group consisting of ethanol, glycerin, propylene glycol, and polyethylene glycol.

22. The stable pharmaceutical formulation of any one of claims 1-21, further comprising a preservative.

23. The stable pharmaceutical formulation of claim 22, wherein the preservative is selected from at least one of the group consisting of benzyl alcohols, methyl parabens and propyl parabens.

24. The stable pharmaceutical formulation of any one of the above claims 1-23 further comprising a thermo-responsive polymer that does not gel at a temperature from about 30°C to about 37°C.

25. The stable pharmaceutical formulation of any one of claims 1-24, wherein said incretin or incretin mimetic is complexed with zinc to form a zinc complex.

26. The stable pharmaceutical formulation of claim 25, wherein the zinc complex comprises a GLP-1 or an analog thereof.

27. The stable pharmaceutical formulation of claim 25, wherein the zinc complex comprises an exendin or an analog thereof.

28. The stable pharmaceutical formulation of claim 25, wherein the zinc complex comprises an exendin-4 zinc complex.

29. The stable pharmaceutical formulation of any one of claims 25-28, wherein the zinc complex is dispersed in the solvent.

30. The stable pharmaceutical formulation of any one of claims 1-29, wherein the formulation has a viscosity of from about 0.25 cP to about 1,000,000 cP.

31. The stable pharmaceutical formulation of any one of claims 1-30, wherein the formulation has a pH at or below the pI of the incretin or incretin mimetic.

32. The stable pharmaceutical formulation of any one of claims 1-30, wherein the formulation has a pH of from about pH 4.0 to about pH 7.5.

33. The stable pharmaceutical formulation of any one of claims 1-30, wherein the formulation has a pH of from about pH 4.0 to about pH 6.0.

34. The stable pharmaceutical formulation of claim 33, wherein the formulation has a pH of about 4.5.

35. The stable pharmaceutical formulation of any one of claims 1-34, wherein the incretin or incretin mimetic is present at a concentration from about 0.1 mg/ml up to the solubility limit of the incretin peptide in the formulation.

36. The stable pharmaceutical formulation of any one of claims 1-34, wherein the incretin or incretin mimetic is present at a concentration from about 1 mg/ml to about 100 mg/ml.

37. The stable pharmaceutical formulation of any one of claims 1-36, wherein said formulation is further lyophilized.

38. The stable pharmaceutical formulation of claim 37, wherein the lyophilized formulation is reconstituted prior to use.

39. The stable pharmaceutical formulation of any one of claims 37-38, wherein the peptide was lyophilized from a solution with a pH ranging from about pH 4 to about pH 6.

40. The pharmaceutical formulation of any one of claims 1-39 for use in treatment of a disease, condition or disorder, wherein the disease, condition or disorder comprises glucose intolerance or diabetes mellitus.

41. The use of the pharmaceutical formulation of any one of claims 1-39 for the preparation of a medicament for treatment of a disease, condition or disorder that may be treated, alleviated or prevented by administering an incretin or an incretin mimetic, wherein the disease, condition or disorder comprises glucose intolerance or diabetes mellitus.

42. The use of claim 41, wherein the disease, condition or disorder is diabetes mellitus.

43. The use of claim 42, wherein the disease, condition or disorder is type 2 diabetes.

44. The use of any one of claims 41-43, wherein the administration is parenteral administration.

45. The use of claim 44, wherein said administration is continuous administration.

46. The use of claim 45, wherein said administration is accomplished via use of an implantable or attachable pump drug delivery device.

47. The use of claim 45 or claim 46, wherein said administration is continuous for a period ranging from about 1 month to about 6 months.

48. The use of claim 44, wherein said administration is accomplished via use of a pen injection device.

49. The use of a stabilizing excipient to improve stability of a pharmaceutical formulation comprising an incretin or incretin mimetic peptide and at least one aprotic, polar solvent, wherein the stabilizing excipient is selected from the group consisting of: water, a sugar, and a sugar alcohol.

50. The use of claim 49, wherein the at least one stabilizing excipient is capable of depressing the freezing point of the aprotic, polar solvent to about 0°C or below.

## Patentansprüche

1. Eine stabile pharmazeutische Formulierung, umfassend ein Inkretin oder Inkretin mimetisches Peptid und mindestens ein aprotisches polares Lösungsmittel und mindestens einen stabilisierenden Hilfsstoff ausgewählt aus der Gruppe bestehend aus: Wasser, einem Zucker und einem Zuckeralkohol.

2. Die stabile pharmazeutische Formulierung nach Anspruch 1, **dadurch gekennzeichnet, dass** das Inkretin oder Inkretin mimetische Peptid ausgewählt ist aus der Gruppe bestehend aus: einem Glukagon-ähnlichen Peptid 1 (glucagon-like-peptide 1, GLP-1) Peptid und einem Exendin Peptid oder einem Analog davon.

3. Die stabile pharmazeutische Formulierung nach Anspruch 2, **dadurch gekennzeichnet, dass** das Exendin Peptid Exendin-4 oder ein Analog davon ist.

4. Die stabile pharmazeutische Formulierung nach Anspruch 1, **dadurch gekennzeichnet, dass** der stabilisierende Hilfsstoff Wasser ist.

5. Die stabile pharmazeutische Formulierung nach einem der Ansprüche 1 - 4, **dadurch gekennzeichnet, dass** das mindestens eine aprotische polare Lösungsmittel ausgewählt ist aus der Gruppe bestehend aus Dimethylsulfoxid (DMSO), Dimethylformamid (DMF), Ethylacetat, N-Methylpyrrolidon (NMP), Dimethylacetamid (DMA), Propylencarbonat und Mischungen davon.

6. Die stabile pharmazeutische Formulierung nach Anspruch 5, **dadurch gekennzeichnet, dass** das mindestens eine aprotische polare Lösungsmittel DMSO ist.

7. Die stabile pharmazeutische Formulierung nach Anspruch 1, **dadurch gekennzeichnet, dass** der mindestens eine stabilisierende Hilfsstoff dazu in der Lage ist, den Gefrierpunkt des aprotischen polaren Lösungsmittels auf etwa 0 °C oder darunter zu senken.

8. Die stabile pharmazeutische Formulierung nach Anspruch 7, **dadurch gekennzeichnet, dass** das aprotische polare Lösungsmittel DMSO ist, während der mindestens eine stabilisierende Hilfsstoff, der dazu in der Lage ist, den Gefrierpunkt des aprotischen polaren Lösungsmittels zu senken, Wasser ist, und dass das Wasser und DMSO ein Co-Lösungsmittel bilden, welches 10 % Gewicht/Gewicht Wasser umfasst (0,67 mol Fraktion DMSO).

9. Die stabile pharmazeutische Formulierung nach einem der Ansprüche 1 - 8, **dadurch gekennzeichnet, dass** der mindestens eine stabilisierende Hilfsstoff dazu in der Lage ist, die Konformation des Inkretin oder Inkretin mimetischen Peptids zu stabilisieren.

10. Die stabile pharmazeutische Formulierung nach einem der Ansprüche 1 - 9, **dadurch gekennzeichnet, dass** das Inkretin Peptid einen oder mehrere Aminosäurereste umfasst, ausgewählt aus der Gruppe bestehend aus Asparagin, Glutamin, Asparaginsäure, Glutaminsäure, Methionin, Cystein, Tryptophan, Tyrosin, Histidin, Lysin und Arginin, und dass das aprotische polare Lösungsmittel und/oder der mindestens eine stabilisierende Hilfsstoff den Aminosäurerest gegenüber Degradation stabilisiert.

11. Die stabile pharmazeutische Formulierung nach Anspruch 10, **dadurch gekennzeichnet, dass** der Aminosäurerest ausgewählt ist aus der Gruppe bestehend aus: Asparagin und Glutamin, wobei das aprotische polare Lösungsmittel und/oder der mindestens eine stabilisierende Hilfsstoff den Aminosäurerest gegenüber Degradation stabilisiert, indem die Bildung von cyclischem Imid oder anderen Abbauprodukten der Asparagin- und Glutamin-Aminosäurereste reduziert oder verhindert wird.

12. Die stabile pharmazeutische Formulierung nach einem der Ansprüche 1 - 11, ferner umfassend mindestens ein nicht wässriges protisches Lösungsmittel.

13. Die stabile pharmazeutische Formulierung nach Anspruch 12, **dadurch gekennzeichnet, dass** das nicht wässrige protische Lösungsmittel ausgewählt ist aus der Gruppe bestehend aus: Polyethylenglykol (PEG), Propylenglykol (PG), Polyvinylpyrrolidon (PVP), Methoxypropylenglykol (MPEG), Glycerol, Glykofurol und Mischungen davon.

14. Die stabile pharmazeutische Formulierung nach einem der Ansprüche 1 - 13, ferner einen Puffer umfassend.

15. Die stabile pharmazeutische Formulierung nach Anspruch 14, **dadurch gekennzeichnet, dass** der Puffer ein Acetatpuffer ist.

16. Die stabile pharmazeutische Formulierung nach einem der Ansprüche 1 - 15, ferner ein Antioxidans umfassend.

17. Die stabile pharmazeutische Formulierung nach Anspruch 16, **dadurch gekennzeichnet, dass** das Antioxidans ausgewählt ist aus mindestens einem aus der Gruppe bestehend aus: Ascorbinsäure, Cystein, Methionin, Monothioglycerol, Natriumthiosulfat, Sulfite, BHT, BHA, Ascorbylpalmitat, Propylgallat und Vitamin E.

18. Die stabile pharmazeutische Formulierung nach einem der Ansprüche 1 - 17, ferner ein Chelat bildendes Agens umfassend.

19. Die stabile pharmazeutische Formulierung nach Anspruch 18, **dadurch gekennzeichnet, dass** der Chelatbildner ausgewählt ist aus mindestens einem aus der Gruppe bestehend aus EDTA, Weinsäure und Salze davon, Glycerin und Zitronensäure und Salze davon.

20. Die stabile pharmazeutische Formulierung nach einem der Ansprüche 1 - 19, ferner umfassend ein nicht wässriges Lösungsmittel.

21. Die stabile pharmazeutische Formulierung nach Anspruch 20, **dadurch gekennzeichnet, dass** das nicht wässrige Lösungsmittel mindestens eins ist, ausgewählt aus der Gruppe bestehend aus Ethanol, Glycerin, Propylenglykol und Polyethylenglykol ist.

22. Die stabile pharmazeutische Formulierung nach einem der Ansprüche 1 - 21, ferner einen Konservierungsstoff umfassend.

23. Die stabile pharmazeutische Formulierung nach Anspruch 22, **dadurch gekennzeichnet, dass** der Konservierungsstoff ausgewählt ist aus mindestens einem aus der Gruppe bestehend aus Benzylalkoholen, Methylparabenen und Propylparabenen.

24. Die stabile pharmazeutische Formulierung nach einem der obigen Ansprüche 1 - 23, ferner umfassend ein thermoresponsives Polymer, welches bei einer Temperatur von etwa 30°C bis etwa 37°C nicht Gel bildet.

25. Die stabile pharmazeutische Formulierung nach einem der Ansprüche 1 - 24, **dadurch gekennzeichnet, dass** das Inkretin oder Inkretin Mimetikum mit Zink komplexiert ist, um einen Zinkkomplex zu bilden.

26. Die stabile pharmazeutische Formulierung nach Anspruch 25, **dadurch gekennzeichnet, dass** der Zinkkomplex ein GLP-1 oder ein Analog davon umfasst.

27. Die stabile pharmazeutische Formulierung nach Anspruch 25, **dadurch gekennzeichnet, dass** der Zinkkomplex ein Exendin oder ein Analog davon umfasst.

28. Die stabile pharmazeutische Formulierung nach Anspruch 25, **dadurch gekennzeichnet, dass** der Zinkkomplex einen Exendin-4 Zinkkomplex umfasst.

29. Die stabile pharmazeutische Formulierung nach einem der Ansprüche 25 - 28, **dadurch gekennzeichnet, dass** der Zinkkomplex in dem Lösungsmittel dispergiert ist.

30. Die stabile pharmazeutische Formulierung nach einem der Ansprüche 1 - 29, **dadurch gekennzeichnet, dass** die Formulierung eine Viskosität von etwa 0,25 cP bis etwa 1 000 000 cP aufweist.

31. Die stabile pharmazeutische Formulierung nach einem der Ansprüche 1 - 30, **dadurch gekennzeichnet, dass** die Formulierung einen pH bei oder unter dem pI des Inkretins oder Inkretin Mimetikums aufweist.

32. Die stabile pharmazeutische Formulierung nach einem der Ansprüche 1 - 30, **dadurch gekennzeichnet, dass** die Formulierung einen pH von etwa pH 4,0 bis etwa pH 7,5 aufweist.

33. Die stabile pharmazeutische Formulierung nach einem der Ansprüche 1 - 30, **dadurch gekennzeichnet, dass** die Formulierung einen pH von etwa pH 4,0 bis etwa pH 6,0 aufweist.

34. Die stabile pharmazeutische Formulierung nach Anspruch 33, **dadurch gekennzeichnet, dass** die Formulierung einen pH von etwa 4,5 aufweist.

35. Die stabile pharmazeutische Formulierung nach einem der Ansprüche 1 - 34, **dadurch gekennzeichnet, dass** das Inkretin oder Inkretin Mimetikum in einer Konzentration von etwa 0,1 mg/ml bis zu der Löslichkeitsgrenze des Inkretin Peptids in der Formulierung vorliegt.

36. Die stabile pharmazeutische Formulierung nach einem der Ansprüche 1 - 34, **dadurch gekennzeichnet, dass** das Inkretin oder Inkretin Mimetikum in einer Konzentration von etwa 1 mg/ml bis etwa 100 mg/ml vorliegt.

37. Die stabile pharmazeutische Formulierung nach einem der Ansprüche 1 - 36, **dadurch gekennzeichnet, dass** die Formulierung ferner lyophilisiert ist.

38. Die stabile pharmazeutische Formulierung nach Anspruch 37, **dadurch gekennzeichnet, dass** die lyophilisierte Formulierung vor Verwendung rekonstituiert wird.

39. Die stabile pharmazeutische Formulierung nach einem der Ansprüche 37 - 38, **dadurch gekennzeichnet, dass** das Peptid aus einer Lösung mit einem pH in dem Bereich von etwa pH 4 bis etwa pH 6 lyophilisiert wurde.

40. Die pharmazeutische Formulierung nach einem der Ansprüche 1 - 39 zur Verwendung bei Behandlung einer Erkrankung, eines Zustands oder einer Störung, **dadurch gekennzeichnet, dass** die Erkrankung, der Zustand oder die Störung Glukoseintoleranz oder Diabetes Mellitus umfasst.

41. Die Verwendung der pharmazeutischen Formulierung nach einem der Ansprüche 1 - 39 zur Herstellung eines Medikaments zur Behandlung einer Erkrankung, eines Zustands oder einer Störung, welche durch Verabreichung eines Inkretins oder eines Inkretin Mimetikums behandelt, verbessert oder verhindert werden kann, wobei die Erkrankung, der Zustand oder die Störung Glukoseintoleranz oder Diabetes Mellitus umfasst.

42. Die Verwendung nach Anspruch 41, **dadurch gekennzeichnet, dass** die Erkrankung, der Zustand oder die Störung Diabetes Mellitus ist.

43. Die Verwendung nach Anspruch 42, **dadurch gekennzeichnet, dass** die Erkrankung, der Zustand oder die Störung Typ 2 Diabetes ist.

44. Die Verwendung nach einem der Ansprüche 41 bis 43, **dadurch gekennzeichnet, dass** die Verabreichung parenterale Verabreichung ist.

45. Die Verwendung nach Anspruch 44, **dadurch gekennzeichnet, dass** die Verabreichung kontinuierliche Verabreichung ist.

46. Die Verwendung nach Anspruch 45, **dadurch gekennzeichnet, dass** die Verabreichung über Verwendung einer implantierbaren oder befestigbaren Medikamentenverabreichungsvorrichtung mit Pumpe erreicht wird.

47. Die Verwendung nach Anspruch 45 oder Anspruch 46, **dadurch gekennzeichnet, dass** die Verabreichung für einen Zeitraum in dem Bereich von etwa einem Monat bis etwa 6 Monaten kontinuierlich ist.

48. Die Verwendung nach Anspruch 44, **dadurch gekennzeichnet, dass** die Verabreichung über Verwendung einer Stiftinjektionsvorrichtung erreicht wird.

49. Die Verwendung eines stabilisierenden Hilfsstoffes zur Verbesserung der Stabilität einer pharmazeutischen Formulierung, umfassend ein Inkretin oder ein Inkretin mimetisches Peptid und mindestens ein aprotisches polares Lösungsmittel, **dadurch gekennzeichnet, dass** der stabilisierende Hilfsstoff ausgewählt ist aus der Gruppe bestehend aus Wasser, einem Zucker und einem Zuckeralkohol.

50. Die Verwendung nach Anspruch 49, **dadurch gekennzeichnet, dass** der mindestens eine stabilisierende Hilfsstoff dazu in der Lage ist, den Gefrierpunkt des aprotischen polaren Lösungsmittels auf etwa 0 °C oder darunter zu senken.

## Revendications

1. Formulation pharmaceutique stable comprenant un peptide incrétine ou incrétino-mimétique et au moins un solvant polaire aprotique, ainsi qu'au moins un excipient stabilisant choisi dans l'ensemble formé par de l'eau, un sucre et un alcool de sucre.

2. Formulation pharmaceutique stable conforme à la revendication 1, pour laquelle le peptide incrétine ou incrétino-mimétique est choisi dans l'ensemble constitué par un peptide GLP-1 (glucagon-like peptide 1) et un peptide exendine ou un analogue d'un tel peptide.

3. Formulation pharmaceutique stable conforme à la revendication 2, dans laquelle le peptide exendine est de l'exendine-4 ou un analogue de celle-ci.

4. Formulation pharmaceutique stable conforme à la revendication 1, dans laquelle l'excipient stabilisant est de l'eau.

5. Formulation pharmaceutique stable conforme à l'une des revendications 1 à 4, pour laquelle le solvant polaire aprotique au nombre d'au moins un est choisi dans l'ensemble constitué par les suivants : diméthyl-sulfoxyde (DMSO), diméthyl-formamide (DMF), acétate d'éthyle, diméthyl-acétamide (DMA), N-méthyl-pyrrolidone (NMP), carbonate de propylène, et leurs mélanges.

6. Formulation pharmaceutique stable conforme à la revendication 5, dans laquelle ledit solvant polaire aprotique au nombre d'au moins un est du DMSO.

7. Formulation pharmaceutique stable conforme à la revendication 1, dans laquelle l'excipient stabilisant au nombre d'au moins un est capable d'abaisser jusqu'environ 0 °C ou au-dessous le point de congélation du solvant polaire aprotique.

8. Formulation pharmaceutique stable conforme à la revendication 7, dans laquelle le solvant polaire aprotique est du DMSO, l'excipient stabilisant au nombre d'au moins un capable d'abaisser le point de congélation du solvant polaire aprotique est de l'eau, et l'eau et le DMSO constituent un mélange solvant comprenant 10 % en poids d'eau, soit une fraction molaire du DMSO valant 0,67.

9. Formulation pharmaceutique stable conforme à l'une des revendications 1 à 8, dans laquelle l'excipient stabilisant au nombre d'au moins un est capable de stabiliser la conformation du peptide incrétine ou incrétino-mimétique.

10. Formulation pharmaceutique stable conforme à l'une des revendications 1 à 9, dans laquelle le peptide incrétine comporte un ou plusieurs résidus d'acides aminés choisis dans l'ensemble formé par les asparagine, glutamine, acide aspartique, acide glutamique, méthionine, cystéine, tryptophane, tyrosine, histidine, lysine et arginine, et le solvant polaire aprotique et/ou l'excipient stabilisant au nombre d'au moins un stabilise(nt) ce ou ces résidu(s) d'acide(s) aminé(s) contre sa ou leur dégradation.

11. Formulation pharmaceutique stable conforme à la revendication 10, dans laquelle le résidu d'acide aminé est choisi parmi l'asparagine et la glutamine, et le solvant polaire aprotique et/ou l'excipient stabilisant au nombre d'au moins un stabilise(nt) ce résidu d'acide aminé contre sa dégradation en empêchant ou réduisant la formation d'imides cycliques ou d'autres produits de dégradation des résidus d'acides aminés asparagine et glutamine.

12. Formulation pharmaceutique stable conforme à l'une des revendications 1 à 11, qui comprend en outre au moins un solvant protique non-aqueux.

13. Formulation pharmaceutique stable conforme à la revendication 12, pour laquelle le solvant protique non-aqueux est choisi dans l'ensemble formé par les suivants : polyéthylène-glycol (PEG), propylène-glycol (PG), poly(vinyl-pyrrolidone) (PVP), méthoxy-propylène-glycol (MPEG), glycérol et glycofurol, ainsi que leurs mélanges.

14. Formulation pharmaceutique stable conforme à l'une des revendications 1 à 13, qui comprend en outre un agent tampon.

15. Formulation pharmaceutique stable conforme à la revendication 14, dans laquelle l'agent tampon est un agent tampon acétate.

16. Formulation pharmaceutique stable conforme à l'une des revendications 1 à 15, qui comprend en outre un agent anti-oxydant.

17. Formulation pharmaceutique stable conforme à la revendication 16, dans laquelle l'agent anti-oxydant est au moins un agent choisi dans l'ensemble formé par les suivants : acide ascorbique, cystéine, méthionine, monothio-glycérol, thiosulfate de sodium, sulfites, hydroxy-toluène butylé (BHT), hydroxy-anisole butylé (BHA), palmitate d'ascorbyle, gallate de propyle, et vitamine E.

18. Formulation pharmaceutique stable conforme à l'une des revendications 1 à 17, qui comprend en outre un agent chélatant.

19. Formulation pharmaceutique stable conforme à la revendication 18, dans laquelle l'agent chélatant est au moins un agent choisi dans l'ensemble formé par les suivants : acide éthylène-diamine-tétracétique (EDTA), acide tartrique, leurs sels, glycérol, et acide citrique et ses sels.

20. Formulation pharmaceutique stable conforme à l'une des revendications 1 à 19, qui comprend en outre un solvant non-aqueux.

21. Formulation pharmaceutique stable conforme à la revendication 20, dans laquelle le solvant non-aqueux est au moins un solvant choisi dans l'ensemble formé par les suivants : éthanol, glycérol, propylène-glycol et polyéthylène-glycol.

22. Formulation pharmaceutique stable conforme à l'une des revendications 1 à 21, qui comprend en outre un agent conservateur.

23. Formulation pharmaceutique stable conforme à la revendication 22, dans laquelle l'agent conservateur est au moins un agent choisi dans l'ensemble formé par les suivants : alcools benzyliques, méthylparabènes et propyl-parabènes.

24. Formulation pharmaceutique stable conforme à l'une des revendications 1 à 23, qui comprend en outre un polymère thermo-sensible qui ne se gélifie pas à une température d'environ 30 °C à environ 37 °C.

25. Formulation pharmaceutique stable conforme à l'une des revendications 1 à 24, dans laquelle ladite incrétine ou ledit incrétino-mimétique est complexé avec du zinc et forme ainsi un complexe de zinc.

26. Formulation pharmaceutique stable conforme à la revendication 25, dans laquelle le complexe de zinc comprend un peptide GLP-1 ou un analogue de GLP-1.

27. Formulation pharmaceutique stable conforme à la revendication 25, dans laquelle le complexe de zinc comprend une exendine ou un analogue d'exendine.

28. Formulation pharmaceutique stable conforme à la revendication 25, dans laquelle le complexe de zinc comprend un complexe de zinc et d'exendine-4.

29. Formulation pharmaceutique stable conforme à l'une des revendications 25 à 28, dans laquelle le complexe de zinc se trouve dispersé dans le solvant.

30. Formulation pharmaceutique stable conforme à l'une des revendications 1 à 29, laquelle formulation présente une viscosité d'environ 0,25 centipoise à environ 1 million de centipoises.

31. Formulation pharmaceutique stable conforme à l'une des revendications 1 à 30, laquelle formulation présente un pH qui est inférieur ou égal au pI de l'incrétine ou de l'incrétino-mimétique.

32. Formulation pharmaceutique stable conforme à l'une des revendications 1 à 30, laquelle formulation présente un pH qui vaut d'environ 4,0 à environ 7,5.

33. Formulation pharmaceutique stable conforme à l'une des revendications 1 à 30, laquelle formulation présente un pH qui vaut d'environ 4,0 à environ 6,0.

34. Formulation pharmaceutique stable conforme à la revendication 33, laquelle formulation présente un pH d'environ 4,5.

35. Formulation pharmaceutique stable conforme à l'une des revendications 1 à 34, dans laquelle l'incrétine ou l'incrétino-mimétique est présent en une concentration valant depuis environ 0,1 mg/mL jusqu'à la limite de solubilité du peptide de type incrétine dans la formulation.

36. Formulation pharmaceutique stable conforme à l'une des revendications 1 à 34, dans laquelle l'incrétine ou l'incrétino-mimétique est présent en une concentration d'environ 1 mg/mL à environ 100 mg/mL.

37. Formulation pharmaceutique stable conforme à l'une des revendications 1 à 36, laquelle formulation est en outre lyophilisée.

38. Formulation pharmaceutique stable conforme à la revendication 37, laquelle formulation lyophilisée est reconstituée avant usage.

39. Formulation pharmaceutique stable conforme à l'une des revendications 37 et 38, dans laquelle le peptide a été lyophilisé à partir d'une solution dont le pH valait d'environ 4 à environ 6.

40. Formulation pharmaceutique stable conforme à l'une des revendications 1 à 39, pour utilisation dans le traitement d'une maladie, d'un état ou d'un trouble, lequel trouble, état ou maladie comprend une intolérance au glucose ou un diabète sucré.

41. Utilisation d'une formulation pharmaceutique stable conforme à l'une des revendications 1 à 39, en vue de la préparation d'un médicament conçu pour le traitement d'une maladie, d'un état ou d'un trouble qui peut être traité, atténué ou prévenu par l'administration d'une incrétine ou d'un incrétino-mimétique, lequel trouble, état ou maladie comprend une intolérance au glucose ou un diabète sucré.

42. Utilisation conforme à la revendication 41, ledit trouble, état ou maladie étant un diabète sucré.

43. Utilisation conforme à la revendication 42, ledit trouble, état ou maladie étant un diabète de type 2.

44. Utilisation conforme à l'une des revendications 41 à 43, ladite administration étant une administration par voie parentérale.

45. Utilisation conforme à la revendication 44, ladite administration étant une administration continue.

46. Utilisation conforme à la revendication 45, ladite administration étant réalisée grâce à un dispositif de délivrance de médicament avec pompe implantable ou attachable.

47. Utilisation conforme à la revendication 45 ou 46, ladite administration étant continue sur une période durant d'environ 1 mois à environ 6 mois.

48. Utilisation conforme à la revendication 44, ladite administration étant réalisée grâce à un dispositif de type stylo à injection.

49. Utilisation d'un excipient stabilisant pour améliorer la stabilité d'une formulation pharmaceutique qui comprend un peptide incrétine ou incrétino-mimétique et au moins un solvant polaire aprotique, lequel excipient stabilisant est choisi dans l'ensemble formé par de l'eau, un sucre et un alcool de sucre.

50. Utilisation conforme à la revendication 49, l'excipient stabilisant au nombre d'au moins un étant capable d'abaisser jusqu'environ 0 °C ou au-dessous le point de congélation du solvant polaire aprotique.
